(19) Europäisches Patentamt
European Patent Office
Office européen des brevets

(11) EP 4 796 216 A1

(12) **EUROPEAN PATENT APPLICATION**

published in accordance with Art. 153(4) EPC

(43) Date of publication:
**26.08.2026 Bulletin 2026/35**

(21) Application number: **24878849.9**

(22) Date of filing: **30.09.2024**

(51) International Patent Classification (IPC):
***B01J 29/072*** *(2006.01)*

(52) Cooperative Patent Classification (CPC):
**B01J 29/00; B01J 29/06; B01J 29/072; B01J 29/70; B01J 37/04; C01B 39/04; C07D 307/36; C07D 307/50**

(86) International application number:
**PCT/CN2024/122732**

(87) International publication number:
**WO 2025/082205 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 CN 202311361864**
**19.10.2023 CN 202311359263**

(71) Applicants:
- **China Petroleum & Chemical Corporation**
  **Beijing 100728 (CN)**
- **Sinopec (Shanghai) Research Institute of Petrochemical Technology Co., Ltd.**
  **Shanghai 201208 (CN)**

(72) Inventors:
- **YANG, Weimin**
  **Shanghai 201208 (CN)**
- **HAN, Xiao**
  **Shanghai 201208 (CN)**
- **LI, Xiangcheng**
  **Shanghai 201208 (CN)**
- **WANG, Zhendong**
  **Shanghai 201208 (CN)**
- **FENG, Xinqiang**
  **Shanghai 201208 (CN)**
- **LIU, Chuang**
  **Shanghai 201208 (CN)**
- **QU, Hongyu**
  **Shanghai 201208 (CN)**

(74) Representative: **Hoffmann Eitle**
**Patent- und Rechtsanwälte PartmbB**
**Arabellastraße 30**
**81925 München (DE)**

# (54) METHOD FOR PREPARING 2,5-DIMETHYLFURAN BY BIOMASS CONVERSION

(57) The present disclosure is directed to a method for preparing 2,5-dimethylfuran by biomass conversion. The method comprises: (1) in an organic solvent, subjecting a biomass feedstock to a reaction in the presence of a solid acid catalyst to obtain a reaction product liquid containing 5-hydroxymethylfurfural and 5-(C2-C5 alkoxymethyl)furfural, wherein the molar ratio of 5-hydroxymethylfurfural to 5-(C2-C5 alkoxymethyl)furfural is in a range of 0.5-1.5; (2) subjecting the reaction product liquid obtained in step (1) and hydrogen to a reaction in the presence of a hydrogenolysis catalyst to prepare 2,5-dimethylfuran. The method has the advantages of high product selectivity, good catalyst stability, and easy product separation, achieving a green and efficient conversion of biomass to 2,5-dimethylfuran.

Solvent Recycling
Hydrogenolysis
Catalyst Loading
Biomass
Feedstock
Solvent I
Solvent II
Acid Catalyst
2
3
4
1
Catalyst Regeneration and Recycling
1、Tank Reactor
2、Solid-Liquid Separator
3、Fixed-Bed Reactor
4、Distillation Unit
Bio-Based
2,5-Dimethylfuran
(DMF)

Fig.1

EP 4 796 216 A1

## Description

### TECHNICAL FIELD

The present disclosure belongs to the field of catalytic conversion of biomass. Specifically, the present disclosure relates to a method for preparing 2,5-dimethylfuran by biomass conversion.

### BACKGROUND ART

With the advancement of science and technology and the development of society, human demand for traditional fossil energy sources such as coal, petroleum, and natural gas is increasing, leading to the gradual depletion of the earth's fossil energy reserves. Moreover, the combustion of fossil energy sources has a serious impact on the environment. In contrast, biomass, as a green and renewable energy source, is abundantly available on earth, and its conversion and utilization process does not pollute the environment. Therefore, many researchers are focusing on using biomass to supplement fossil energy. 2,5-Dimethylfuran (DMF), as an important product of biomass chemical conversion, has promising application prospects. It can be used as a fuel, possessing a high energy density (31.5 MJ/L) and octane number (119). Compared with traditional bioethanol, DMF has significant advantages and is more suitable as a gasoline additive. At the same time, due to the presence of diene bonds on its furan ring, it can also undergo Diels-Alder reactions (D-A reaction) with monoolefins such as ethylene to directly synthesize bio-based para-xylene (p-Xylene, PX).

2,5-Dimethylfuran is mainly prepared by hydrogenolysis of 5-hydroxymethylfurfural (HMF) under the catalysis of noble metals or non-noble metals. Zu Yanhong et al. developed a Ru/$Co_3O_4$ catalyst for the hydrogenolysis of HMF to prepare DMF. Under relatively mild conditions (130°C, 0.7 MPa), the conversion of HMF exceeded 99%, achieving a DMF yield of 93.4% (Appl. Catal. B: Environ. [J]. 2014, 146, 244-248). Current production processes for 2,5-dimethylfuran often suffer from problems such as high raw material costs and short catalyst life, leading to high prices of DMF and making large-scale application difficult. CN103554066A reports a strategy for preparing 2,5-dimethylfuran starting from fructose, using a nickelditungsten carbide catalyst supported on activated carbon. First, a tetrahydrofuran system containing sulfuric acid was used to carry out a dehydration reaction at 160°C to prepare 5-hydroxymethylfurfural, with a maximum yield of 64%. Subsequently, under the action of the nickelditungsten carbide catalyst supported on activated carbon, DMF was prepared, with a maximum yield of 95%. CN105175366A reports a one-step method for producing 2,5-dimethylfuran, wherein fructose was used as the raw material, a mixed solution of Ru/C, $AlCl_3$ and inorganic acid was used as the catalyst, and N,N'-dimethylformamide was used as the solvent. The total yield of DMF could reach up to 66.3%. The above preparation methods all involve the use of liquid acids. Although the catalytic effect is excellent, the use of liquid acids causes problems such as severe equipment corrosion, significant environmental pollution due to the waste liquid from alkali neutralization treatment, and many side reactions. At the same time, a large amount of by-products (such as humins) are generated, which affects the separation of the product and the stability of the hydrogenolysis catalyst. Moreover, the substrate concentration in the reaction for HMF hydrogenolysis to generate DMF currently reported is relatively low, and the process for obtaining pure DMF would significantly increase separation costs. Therefore, there is a need for a green and efficient method for preparing 2,5-dimethylfuran by biomass conversion, which can efficiently convert biomass feedstocks such as fructose and glucose into 2,5-dimethylfuran.

### SUMMARY

The technical problem to be solved by the present disclosure is the problems existing in the prior art for the preparation process of 2,5-dimethylfuran, such as low production efficiency, high cost, and environmental pollution. To solve the above technical problems, the present disclosure provides a green and efficient method for preparing 2,5-dimethylfuran by biomass conversion. This method is simple to operate and has the advantages of high product selectivity, good catalyst stability, and easy product separation, achieving a green and efficient conversion of biomass to 2,5-dimethylfuran.

The present disclosure provides a method for preparing 2,5-dimethylfuran by biomass conversion, comprising:

(1) in an organic solvent, subjecting a biomass feedstock to a reaction in the presence of a solid acid catalyst to obtain a reaction product liquid containing 5-hydroxymethylfurfural (HMF) and 5-(C2-C5 alkoxymethyl)furfural (RMF), wherein the molar ratio of 5-hydroxymethylfurfural to 5-(C2-C5 alkoxymethyl)furfural is in a range of 0.5-1.5;
(2) subjecting the reaction product liquid obtained in step (1) and hydrogen to a reaction in the presence of a hydrogenolysis catalyst to prepare 2,5-dimethylfuran.

In some embodiments, in step (1), the organic solvent comprises solvent I and solvent II; the solvent I is selected from at least one of C2-C5 alcohols, preferably selected from at least one of ethanol, n-propanol, n-butanol, isobutanol,

isopropanol, isoamyl alcohol, ethylene glycol, propylene glycol and glycerol, more preferably selected from one or two of isopropanol, isoamyl alcohol and n-butanol; the solvent II is selected from at least one of cyclic ether compounds, methyl isobutyl ketone and γ-valerolactone, the cyclic ether compound is preferably selected from at least one of pyran, 2-methyltetrahydrofuran, 2,5-dimethyltetrahydrofuran, tetrahydrofuran and 1,4-dioxane, and the solvent II is preferably selected from one or two of 2,5-dimethyltetrahydrofuran, 1,4-dioxane and tetrahydrofuran. In some embodiments, the mass ratio of the solvent I to the solvent II is in a range of 0.1-15:1, preferably 0.5-10:1. In some embodiments, the mass ratio of the total amount of the organic solvent to the biomass feedstock is in a range of 1-100:1, preferably 2-50: 1.

In some embodiments, in step (1), the biomass feedstock is a biomass feedstock containing hexose and/or a biomass feedstock that produces hexose upon treatment, wherein the hexose is selected from one or more of galactose, mannose, glucose and fructose, preferably fructose and/or glucose. In some embodiments, in step (1), the mass ratio of the biomass feedstock to the solid acid catalyst is in a range of 0.2-20:1, preferably 0.4-20:1. In some embodiments, in step (1), the reaction temperature is in a range of 90-220°C, preferably 120-200°C; the reaction time is in a range of 5-300 min, preferably 10-240 minutes. In some embodiments, the reaction in step (1) is carried out under an inert gas, and the inert gas pressure is in a range of 0.1-3.0 MPa, preferably 0.5-2.0 MPa. In some embodiments, the solid acid catalyst in step (1) is selected from one or more of SCM-36-$SO_3H$ molecular sieve, SCM-1-$SO_3H$ molecular sieve, H-beta molecular sieve, USY molecular sieve, SBA-15-$SO_3H$ molecular sieve, sulfonated carbon, A-15 resin or Aquivion@$SiO_2$ resin; preferably SCM-36-$SO_3H$ molecular sieve.

In some embodiments, in step (2), the mass ratio of the reaction product liquid containing 5-hydroxymethylfurfural and 5-(C2-C5 alkoxymethyl)furfural obtained in step (1) to the hydrogenolysis catalyst is in a range of 30-200: 1, preferably 50-150: 1. In some embodiments, in step (2), the hydrogenolysis catalyst is a supported noble metal and/or non-noble metal catalyst, preferably selected from one or more of Pd/C, Pt/C, Ru/C, Pd/$Al_2O_3$, Pt/$Al_2O_3$, Ru/$Al_2O_3$, Ru/$Co_3O_4$, Ni-$Co_3O_4$/C, Ni/$Al_2O_3$, Ni-Fe/C, Ni-$C_3N_4$/HC catalyst and Ni-SCM-X molecular sieve catalyst; more preferably selected from Ni-SCM-X molecular sieve catalyst and Ni-$C_3N_4$/HC catalyst. In some embodiments, the reaction in step (2) is carried out under a hydrogen atmosphere, and the hydrogen pressure is in a range of 0.5-4.0 MPa, preferably 1-3.0 MPa; and/or, the reaction temperature is in a range of 130-220°C, preferably 150-200°C; the reaction time is in a range of 1-12 h, preferably 2-10 h.

In some embodiments, the step (1) further comprises filtering a mixture produced by the reaction to remove the solid acid catalyst to obtain the reaction product liquid, preferably the reaction product liquid obtained in step (1) is directly used in step (2).

Compared with the prior art, the present disclosure has the following beneficial effects:

1) In the present disclosure, biomass is used as a raw material, which is inexpensive and widely available. By employing a functional group protection strategy, the chemically active, unstable, and difficult-to-store intermediate product HMF is selectively passivated (etherified) in one step, partially producing RMF. This reduces the self-polymerization of HMF and the formation of other by-products to a certain extent, increasing the substrate concentration while ensuring a high yield of the target product.

2) In the present disclosure, the reaction product liquid containing HMF and RMF produced in step (1) can be directly used for the hydrogenolysis reaction in step (2), undergoing the cleavage of carbon-oxygen bonds while the protecting groups are removed, to generate DMF. This solves the problem of generally low carbon yield in the reaction for producing DMF from hydrogenolysis of HMF at a high concentration, and also avoids the high separation energy consumption caused by excessively low product concentration.

3) In the present disclosure, the product DMF is finally obtained through a tandem full-process flow of biomass → RMF(HMF) and RMF(HMF) → DMF. The same mixed organic solvent system is used in both reaction steps, without involving complex separation processes of HMF or RMF or solvent replacement processes in between. The protection of the reactive group and the removal of the protecting group are achieved during the tandem two-step reaction process. The present disclosure further maximizes the selectivity for the final product DMF by adjusting the molar ratio of HMF/RMF in the reaction product liquid obtained in step (1).

4) The present disclosure optimizes the design of catalysts for the different reactions in steps (1) and (2). In a preferable embodiment, a modified molecular sieve based on the new SCM series structure is used as the catalyst. In the biomass → RMF(HMF) reaction, a modified SCM-36-$SO_3H$ catalyst, obtained by loading sulfonate on hydrogen-form SCM-36 which has a relatively large number of silanol groups on the surface, is preferably used to catalyze the dehydration reaction. In the RMF(HMF) → DMF reaction, a catalyst obtained by loading a non-noble metal onto a silicon-germanium molecular sieve SCM-14 or SCM-15 is preferably used to catalyze selective hydrogenation. In another preferable embodiment, a catalyst obtained by loading a non-noble metal and a nitrogen-containing precursor onto activated carbon activated with hydrogen is used to catalyze selective hydrogenation. By designing specific catalysts for different reaction steps, the substrate conversion and product selectivity under mild reaction conditions are further improved.

The present application also provides the following embodiments:

1. A method for preparing 2,5-dimethylfuran by biomass conversion, comprising:

(1) in the presence of multiple organic solvents, bringing a biomass feedstock into contact with a solid acid catalyst to carry out a reaction, so as to obtain a reaction product liquid containing 5-hydroxymethylfurfural and a passivated product with functional groups of 5-hydroxymethylfurfural partially passivated;
(2) bringing the reaction product liquid obtained in step (1) and hydrogen into contact with a hydrogenolysis catalyst to carry out a reaction, so as to produce 2,5-dimethylfuran.

2. The method according to embodiment 1, characterized in that, in step (1), the organic solvents comprise solvent I and solvent II; the solvent I is an alcohol solvent, preferably at least one of methanol, ethanol, n-propanol, n-butanol, isopropanol, isoamyl alcohol, ethylene glycol, propylene glycol, glycerol or octanol, further preferably one or two of isopropanol, isoamyl alcohol or n-butanol; the solvent II is selected from at least one of tetrahydrofuran, 1,4-dioxane, methyl isobutyl ketone, $\gamma$-valerolactone or toluene, preferably one or two of 2,5-dimethyltetrahydrofuran, 1,4-dioxane or tetrahydrofuran;

and/or, the mass ratio of the solvent I to the solvent II is in a range of 0.1-100: 1, preferably 0.5-20:1;
and/or, the mass ratio of the total amount of organic solvents to the biomass feedstock is in a range of 1-100:1, preferably 2-50:1.

3. The method according to embodiment 1, characterized in that, in step (1), the biomass feedstock is one or more of cellulose, inulin, cellobiose, sucrose, glucose or fructose, preferably fructose and/or glucose;
and/or, in step (1), the mass ratio of the biomass feedstock to the solid acid catalyst is in a range of 0.2-20:1, preferably 0.4-20:1.
4. The method according to embodiment 1, characterized in that, in step (1), the reaction temperature is in a range of 90-220°C, preferably 120-200°C; the reaction time is in a range of 5-300 min, preferably 10-240 minutes;
and/or, in step (1), the reaction is carried out under an inert gas, and the inert gas pressure is in a range of 0.1-3.0 MPa, preferably 0.5-2.0 MPa.
5. The method according to embodiment 1, characterized in that, in step (1), the solid acid catalyst is selected from any one of SCM-36-$SO_3H$ molecular sieve, SCM-1-$SO_3H$ molecular sieve, H-beta molecular sieve, USY molecular sieve, sulfonated carbon or A-15 resin; preferably SCM-36-$SO_3H$ molecular sieve;

preferably, the SCM-36-$SO_3H$ molecular sieve has a Brønsted acid/Lewis acid ratio of 2.2-4.0;
preferably, in the SCM-36-$SO_3H$ molecular sieve, the content of -$SO_3H$ is in a range of 1.9 wt%-18.0 wt%;
preferably, the external specific surface area/total specific surface area ratio of the SCM-36-$SO_3H$ molecular sieve is in a range of 0.25-1, preferably 0.3-0.7.

6. The method according to embodiment 1, characterized in that, in step (2), the hydrogenolysis catalyst is a noble metal and/or non-noble metal hydrogenation catalyst, comprising any one of Pd/C, Pt/C, Ru/C, Pd/$Al_2O_3$, Pt/$Al_2O_3$, Ru/$Al_2O_3$, Ru/$Co_3O_4$, Ni-$Co_3O_4$/C, Ni-$Al_2O_3$, Ni-Fe/C or Ni-SCM-X; preferably Ni-SCM-X.
7. The method according to embodiment 6, characterized in that, in the Ni-SCM-X, X is 14 or 15;

and/or, in the Ni-SCM-X, based on the mass of the SCM-X molecular sieve, the content of Ni, calculated as Ni, is in a range of 0.2 wt%-5.0 wt%, preferably 0.5 wt%-3.0 wt%;
and/or, in the Ni-SCM-X, the Si/Ge molar ratio of the SCM-X molecular sieve is in a range of 1.5-8, preferably 2-7.

8. The method according to embodiment 6 or 7, characterized in that, the total acid amount of the Ni-SCM-X is in a range of 130-220 $\mu mol \cdot g^{-1}$, preferably 150-200 $\mu mol \cdot g^{-1}$;

and/or, the Brønsted acid/Lewis acid ratio of the Ni-SCM-X is in a range of 0.3-0.8, preferably 0.4-0.6;
and/or, the molar ratio of metallic Ni to oxidized NiO in the Ni-SCM-X is in a range of 0.02-0.15, preferably 0.05-0.12;
and/or, the mean particle size of nickel particles in the Ni-SCM-X is in a range of 2-6 nm, preferably 3-5 nm.

9. The method according to embodiment 1, characterized in that, in step (2), the mass ratio of the reaction product liquid containing 5-hydroxymethylfurfural and its passivated product obtained from the reaction in step (1) to the hydrogenolysis catalyst is in a range of 30-200:1, preferably 50-150:1.

10. The method according to embodiment 1, characterized in that, in the reaction system of step (2), hydrogen is charged to adjust the reaction pressure; the reaction pressure is in a range of 0.5-4.0 MPa, preferably 0.5-3.0 MPa; and/or, the reaction conditions in step (2) are as follows: the reaction temperature is in a range of 130-220°C, preferably 150-200°C; the reaction time is in a range of 1-12 h, preferably 2-10 h.

Additional features and advantages of the present application will be described in detail in the subsequent detailed description section.

## BRIEF DESCRIPTION OF THE DRAWINGS

The accompanying drawings are intended to provide a further understanding of the present application, constitute a part of the specification, and together with the following detailed description serve to explain the present application, but do not constitute a limitation on the present application. In the accompanying drawings:

FIG. 1 is a schematic flow diagram of the preparation of 2,5-dimethylfuran by biomass conversion according to the present disclosure;
FIG. 2 shows infrared spectra of hydrogen-form SCM-36 and conventional hydrogen-form FER molecular sieve;
FIG. 3 shows an XRD pattern of the solid acid catalyst SCM-36-$SO_3H$ obtained in Preparation Example 1;
FIG. 4 shows an SEM image of the solid acid catalyst SCM-36-$SO_3H$ obtained in Preparation Example 1;
FIG. 5 shows a pyridine adsorption infrared spectrum of the solid acid catalyst SCM-36-$SO_3H$ obtained in Preparation Example 1;
FIG. 6 shows an XRD pattern of the hydrogenolysis catalyst Ni-SCM-14 obtained in Preparation Example 2;
FIG. 7 shows an $NH_3$-TPD pattern of the hydrogenolysis catalyst Ni-SCM-14 obtained in Preparation Example 2;
FIG. 8 shows an XPS spectrum of the hydrogenolysis catalyst Ni-SCM-14 obtained in Preparation Example 2;
FIG. 9 shows a TEM image and particle size distribution diagram of the hydrogenolysis catalyst Ni-SCM-14 obtained in Preparation Example 2;
FIG. 10 shows an XRD pattern of the hydrogenolysis catalyst Ni-SCM-15 obtained in Preparation Example 3;
FIG. 11 shows an XRD pattern of the hydrogenolysis catalyst Ni-$C_3N_4$/HC obtained in Preparation Example 4;
FIG. 12 shows a TEM image of the hydrogenolysis catalyst Ni-$C_3N_4$/HC obtained in Preparation Example 4;
FIG. 13 shows an XPS spectrum of the hydrogenolysis catalyst Ni-$C_3N_4$/HC obtained in Preparation Example 4.

## DETAILED DESCRIPTION

The specific embodiments of the present application will be described in detail below with reference to the accompanying drawings. It should be understood that the specific embodiments described herein are only used to illustrate and explain the present application and are not intended to limit the present application.

Any specific numerical value (including the endpoints of numerical ranges) disclosed herein is not limited to the precise value but should be understood to also encompass values close to that precise value, such as all possible values within a range of ±5% of that precise value. Furthermore, for disclosed numerical ranges, one or more new numerical ranges can be obtained by arbitrary combination between the endpoint values of the range, between endpoint values and specific point values within the range, and between various specific point values. These new numerical ranges should also be considered as specifically disclosed herein.

Unless otherwise indicated, the terms used herein have the same meanings as commonly understood by a person skilled in the art. If a term is defined herein and the definition differs from the common understanding in the art, the definition herein shall prevail.

As mentioned above, the present disclosure provides a method for preparing 2,5-dimethylfuran by biomass conversion, comprising:

(1) in an organic solvent, subjecting a biomass feedstock to a reaction in the presence of a solid acid catalyst to obtain a reaction product liquid containing 5-hydroxymethylfurfural and 5-(C2-C5 alkoxymethyl)furfural, wherein the molar ratio of 5-hydroxymethylfurfural to 5-(C2-C5 alkoxymethyl)furfural is in a range of 0.5-1.5;
(2) subjecting the reaction product liquid obtained in step (1) and hydrogen to a reaction in the presence of a hydrogenolysis catalyst to prepare 2,5-dimethylfuran.

Dehydration Step (1)

According to the present disclosure, in step (1), a biomass feedstock is subjected to a dehydration reaction in a mixed organic solvent comprising an alcohol solvent and a non-alcohol solvent in the presence of a solid acid catalyst to

obtain a reaction product liquid containing HMF and RMF, wherein RMF is the selectively passivated (etherified) product of HMF. The present inventors have verified through repeated experiments that when the molar ratio of HMF to RMF in the reaction product liquid from step (1) is controlled within the range of 0.5-1.5, preferably 0.6-1.4, the selectivity for 2,5-dimethylfuran in the hydrogenolysis step (2) can be maximized. Without wishing to be bound by any particular theory, it is believed that by selectively passivating (etherifying) the reactive and unstable intermediate product HMF, partially producing RMF, the self-polymerization of HMF and the formation of other by-products are reduced to a certain extent, allowing for an increase in substrate concentration, while ensuring a high yield of the target product by adjusting the activity of the reaction system, and also avoiding the high separation energy consumption caused by excessively low product concentration.

In some embodiments, in step (1), the molar ratio of HMF to RMF may be 0.5, 0.6, 0.7, 0.8, 0.9, 1.0, 1.1, 1.2, 1.3, 1.4, 1.5, or in a range between any two of the above values.

In some embodiments, in step (1), the biomass feedstock is a biomass feedstock containing hexose and/or a biomass feedstock that produces hexose upon treatment. Preferably, the treatment is any treatment method known to those skilled in the art that can degrade polysaccharide biomass (such as straw, including corncob, corn stover, wheat straw, rice straw, etc.; sugar waste, including bagasse, beet pulp, etc.) to generate hexose, particularly hexose selected from one or more of galactose, mannose, glucose and fructose, for example acid hydrolysis or enzymatic hydrolysis, etc. Preferably, the biomass feedstock is a hexose, preferably selected from one or more of galactose, mannose, glucose and fructose, most preferably fructose.

In some embodiments, the initial mass concentration of the biomass feedstock is at least 0.1 wt%, preferably at least 1 wt%, more preferably at least 5 wt%. In some embodiments, the initial mass concentration of the biomass feedstock is at most 40 wt%, preferably at most 35 wt%, more preferably at most 30 wt%. In some embodiments, the initial mass concentration of the biomass feedstock may be 0.1 wt%, 0.5 wt%, 1 wt%, 2 wt%, 3 wt%, 4 wt%, 5 wt%, 6 wt%, 7 wt%, 8 wt%, 9 wt%, 10 wt%, 11 wt%, 12 wt%, 13 wt%, 14 wt%, 15 wt%, 16 wt%, 17 wt%, 18 wt%, 19 wt%, 20 wt%, 21 wt%, 22 wt%, 23 wt%, 24 wt%, 25 wt%, 26 wt%, 27 wt%, 28 wt%, 29 wt%, 30 wt%, 31 wt%, 32 wt%, 33 wt%, 34 wt%, 35 wt%, 36 wt%, 37 wt%, 38 wt%, 39 wt%, 40 wt%, or in a range between any two of the above values.

In some embodiments, in step (1), the organic solvent comprises an alcohol solvent I and a non-alcohol solvent II. Preferably, the solvent I is selected from at least one of C2-C5 alcohols, preferably selected from at least one of ethanol, n-propanol, n-butanol, isobutanol, isopropanol, isoamyl alcohol, ethylene glycol, propylene glycol and glycerol, more preferably selected from one or two of isopropanol, isoamyl alcohol and n-butanol. Preferably, the solvent II is selected from at least one of cyclic ether compounds, methyl isobutyl ketone and γ-valerolactone, the cyclic ether compound is preferably selected from at least one of pyran, 2-methyltetrahydrofuran, 2,5-dimethyltetrahydrofuran, tetrahydrofuran and 1,4-dioxane, and the solvent II is preferably selected from one or two of 2,5-dimethyltetrahydrofuran, 1,4-dioxane and tetrahydrofuran. Preferably, the organic solvent comprises solvent I selected from at least one of C2-C5 alcohols and solvent II selected from at least one of cyclic ether compounds. More preferably, the organic solvent comprises solvent I selected from one or two of isopropanol, isoamyl alcohol and n-butanol, and solvent II selected from one or two of 2,5-dimethyltetrahydrofuran, 1,4-dioxane and tetrahydrofuran. Most preferably, the organic solvent comprises n-butanol and tetrahydrofuran.

In some embodiments, the mass ratio of the solvent I to the solvent II is in a range of 0.1-15:1, preferably 0.5-10:1. In some embodiments, the mass ratio of the solvent I to the solvent II may be 0.1:1, 0.2:1, 0.5:1, 0.8:1, 1:1, 2:1, 3:1, 4:1, 5:1, 6:1, 7:1, 8:1, 9:1, 10:1, 11:1, 12:1, 13:1, 14:1, 15:1, or in a range between any two of the above values.

In some embodiments, the mass ratio of the total amount of the organic solvent to the biomass feedstock is in a range of 1-100:1, preferably 2-50:1. In some embodiments, the mass ratio of the total amount of the organic solvent to the biomass feedstock may be 1:1, 2:1, 3:1, 4:1, 5:1, 6:1, 7:1, 8:1, 9:1, 10:1, 15:1, 20:1, 25:1, 30:1, 35:1, 40:1, 45:1, 50:1, 55:1, 60:1, 65:1, 70:1, 75:1, 80:1, 85:1, 90:1, 95:1, 100:1, or in a range between any two of the above values.

In some embodiments, the mass ratio of the biomass feedstock to the solid acid catalyst is in a range of 0.2-20:1, preferably 0.4-20:1. In some embodiments, the mass ratio of the biomass feedstock to the solid acid catalyst may be 0.2:1, 0.3:1, 0.4:1, 0.5:1, 0.6:1, 0.7:1, 0.8:1, 0.9:1, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, or in a range between any two of the above values.

In some embodiments, in step (1), the reaction temperature is in a range of 90-220°C, preferably 120-200°C, more preferably 150-180°C, for example, may be 90°C, 100°C, 110°C, 120°C, 130°C, 140°C, 150°C, 160°C, 170°C, 180°C, 190°C, 200°C, or in a range between any two of the above values; the reaction time is in a range of 5-300 min, preferably 10-240 min, for example, may be 5 min, 10 min, 50 min, 80 min, 100 min, 120 min, 150 min, 180 min, 200 min, 240 min, 300 min, or in a range between any two of the above values.

In some embodiments, in step (1), the reaction is carried out under an inert gas, and the inert gas pressure is in a range of 0.1-3.0 MPa, preferably 0.5-2.0 MPa, for example, may be 0.1 MPa, 0.2 MPa, 0.4 MPa, 0.6 MPa, 0.8 MPa, 1 MPa, 1.5 MPa, 2 MPa, 2.5 MPa, 3.0 MPa, or in a range between any two of the above values. The type of inert gas is not particularly limited and may be conventional choices in the art. Preferably, the inert gas is nitrogen, argon, or a mixture thereof.

In some embodiments, in step (1), the solid acid catalyst is selected from one or more of SCM-36-$SO_3H$ molecular sieve, SCM-1-$SO_3H$ molecular sieve, H-beta molecular sieve, USY molecular sieve, SBA-15-$SO_3H$ molecular sieve, sulfonated carbon, A-15 resin or Aquivion@$SiO_2$ resin. Preferably, the solid acid catalyst is SCM-36-$SO_3H$ molecular sieve.

SCM-36-$SO_3H$ Molecular Sieve Catalyst

In some embodiments, the solid acid catalyst is an SCM-36-$SO_3H$ molecular sieve having a schematic chemical composition represented by the formula "$mSiO_2 \bullet nAl_2O_3 \bullet pSO_3H$", wherein $22 \leq m/n \leq 80$, and $10 \leq m/p \leq 160$; preferably, $25 \leq m/n \leq 66$, $25 \leq m/p \leq 120$; more preferably, $25 \leq m/n \leq 60$, $26 \leq m/p \leq 80$. For example, m/n may be 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or in a range between any two of the above values; for example, m/p may be 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, or in a range between any two of the above values. In the present disclosure, the values of m/n and m/p can be obtained by measuring elemental contents using inductively coupled plasma atomic emission spectrometry and then calculating.

In some embodiments, the SCM-36-$SO_3H$ molecular sieve is obtained by subjecting a hydrogen-form SCM-36 molecular sieve to sulfonation modification, wherein the silica-alumina ratio of the hydrogen-form SCM-36 molecular sieve is in a range of 20-50, for example, may be 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or in a range between any two of the above values; the ratio of terminal silanol groups to the sum of internal silanol groups and bridging hydroxyl groups in the hydrogen-form SCM-36 molecular sieve is in a range of 0.2-1.2, for example, may be 0.2, 0.3, 0.4, 0.5, 0.6, 0.7, 0.8, 0.9, 1.0, 1.1, 1.2, or in a range between any two of the above values.

The SCM-36 molecular sieve and the preparation method thereof are described in CN115959681A, the entire contents of which are incorporated herein by reference.

In some embodiments, the Brønsted acid/Lewis acid ratio of the SCM-36-$SO_3H$ molecular sieve is in a range of 2.0-4.5, preferably 2.2-4.0. In some embodiments, the Brønsted acid/Lewis acid ratio of the SCM-36-$SO_3H$ molecular sieve may be 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, or in a range between any two of the above values. In the present disclosure, the acid type and acid amount can be determined by pyridine adsorption infrared spectroscopy, and the Brønsted acid/Lewis acid ratio can be calculated.

In some embodiments, in the SCM-36-$SO_3H$ molecular sieve, the content of -$SO_3H$ is in a range of 1.5 wt%-20.0 wt%, preferably 1.9 wt%-18.0 wt%. In some embodiments, the content of -$SO_3H$ may be 1.5 wt%, 1.6 wt%, 1.7 wt%, 1.8 wt%, 1.9 wt%, 2.0 wt%, 2.5 wt%, 3.0 wt%, 3.5 wt%, 4.0 wt%, 4.5 wt%, 5.0 wt%, 5.5 wt%, 6.0 wt%, 6.5 wt%, 7.0 wt%, 7.5 wt%, 8.0 wt%, 8.5 wt%, 9.0 wt%, 9.5 wt%, 10.0 wt%, 10.5 wt%, 11.0 wt%, 11.5 wt%, 12.0 wt%, 12.5 wt%, 13.0 wt%, 13.5 wt%, 14.0 wt%, 14.5 wt%, 15.0 wt%, 15.5 wt%, 16.0 wt%, 16.5 wt%, 17.0 wt%, 17.5 wt%, 18.0 wt%, 18.5 wt%, 19.0 wt%, 19.5 wt%, 20.0 wt%, or in a range between any two of the above values. In the present disclosure, the content of -$SO_3H$ can be obtained by measuring the S element content using inductively coupled plasma atomic emission spectrometry and then calculating.

In some embodiments, the SCM-36-$SO_3H$ molecular sieve is an SCM-36 molecular sieve having -$SO_3H$ groups, wherein the SCM-36 molecular sieve has a schematic chemical composition represented by the formula "$mSiO_2 \bullet nAl_2O_3$", wherein $22 \leq m/n \leq 80$, preferably $25 \leq m/n \leq 66$, more preferably $25 \leq m/n \leq 60$, wherein the content of -$SO_3H$ groups is in a range of 1.5 wt%-20.0 wt%, preferably 1.9 wt%-18.0 wt%, based on the weight of the modified SCM-36 molecular sieve. In some embodiments, m/n may be 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or in a range between any two of the above values; the content of -$SO_3H$ groups may be 1.5 wt%, 1.6 wt%, 1.7 wt%, 1.8 wt%, 1.9 wt%, 2.0 wt%, 2.5 wt%, 3.0 wt%, 3.5 wt%, 4.0 wt%, 4.5 wt%, 5.0 wt%, 5.5 wt%, 6.0 wt%, 6.5 wt%, 7.0 wt%, 7.5 wt%, 8.0 wt%, 8.5 wt%, 9.0 wt%, 9.5 wt%, 10.0 wt%, 10.5 wt%, 11.0 wt%, 11.5 wt%, 12.0 wt%, 12.5 wt%, 13.0 wt%, 13.5 wt%, 14.0 wt%, 14.5 wt%, 15.0 wt%, 15.5 wt%, 16.0 wt%, 16.5 wt%, 17.0 wt%, 17.5 wt%, 18.0 wt%, 18.5 wt%, 19.0 wt%, 19.5 wt%, 20.0 wt%, or in a range between any two of the above values, based on the weight of the modified SCM-36 molecular sieve. In the present disclosure, the value of m/n and the content of -$SO_3H$ groups can be obtained by measuring elemental contents using inductively coupled plasma atomic emission spectrometry and then calculating.

Preferably, the external specific surface area/total specific surface area ratio of the SCM-36-$SO_3H$ molecular sieve is in a range of 0.25-0.9, preferably 0.25-0.7. For example, the external specific surface area/total specific surface area ratio of the SCM-36-$SO_3H$ molecular sieve may be 0.25, 0.30, 0.35, 0.40, 0.45, 0.50, 0.55, 0.60, 0.65, 0.70, 0.75, 0.80,

0.85, 0.90, or in a range between any two of the above values. In the present disclosure, the specific surface area can be measured by the BET method, and then the external specific surface area/total specific surface area ratio can be obtained by calculation.

In some embodiments, the method for preparing the SCM-36-$SO_3H$ molecular sieve comprises: subjecting a hydrogen-form SCM-36 molecular sieve to acid elution, addition of a mercapto compound, oxidation, and hydrothermal treatment. The hydrogen-form SCM-36 molecular sieve can be prepared according to the method described in CN115959681A, the entire contents of which are incorporated herein by reference.

In some embodiments, the method for preparing the SCM-36-$SO_3H$ molecular sieve comprises the following steps:

S1: mixing a silicon source, an aluminum source, an alkali source, an organic structure-directing agent, and water to obtain a mixture, then subjecting the mixture to crystallization, a first calcination, ion exchange, and a second calcination to obtain a hydrogen-form SCM-36 molecular sieve;

S2: dispersing the hydrogen-form SCM-36 molecular sieve in a solvent, adding a mercapto compound to form a reaction solution A; filtering the reaction solution A, followed by washing under reflux, to obtain a solid B;

S3: mixing the solid B, an oxidizing agent, an alcohol, and water to obtain a suspension; filtering the suspension, followed by post-treatment, to obtain a modified SCM-36-$SO_3H$ molecular sieve.

In some embodiments, the SCM-36 molecular sieve as-synthesized is calcined to remove the structure-directing agent, then subjected to ion exchange and calcination to obtain a hydrogen-form SCM-36 molecular sieve. Herein, calcination and ion exchange are conventional operations in the art. Preferably, the hydrogen-form SCM-36 molecular sieve has a silica-alumina ratio in a range of 20-50; the ratio of terminal silanol groups to the sum of internal silanol groups and bridging hydroxyl groups is in a range of 0.2-1.2.

In some embodiments, the type of silicon source is not particularly limited and can be conventional choices in the art. Preferably, the silicon source is at least one of silica sol and silica gel.

In some embodiments, the type of aluminum source is not particularly limited and can be conventional choices in the art. Preferably, the aluminum source is an aluminate, such as sodium aluminate.

In some embodiments, the type of alkali source is not particularly limited and can be conventional choices in the art. Preferably, the alkali source is sodium hydroxide.

In some embodiments, the organic structure-directing agent contains a trimethylethylammonium ion structure.

In some embodiments, the aluminum source is sodium aluminate, wherein the content of $Al_2O_3$ is in a range of 38 wt%-43 wt%, and the content of $Na_2O$ is in a range of 30 wt%-33 wt%.

In some embodiments, in the mixture of step S1, the molar ratio of the silicon source (calculated as $SiO_2$), the aluminum source (calculated as $Al_2O_3$), the alkali source (calculated as $OH^-$), the organic structure-directing agent (calculated as trimethylethylammonium ion), and water is in a range of 1 : 0.016-0.050 : 0.10-0.20 : 0.15-0.30 : 10-50.

In some embodiments, the crystallization conditions for the mixture of step S1 include: crystallization temperature: 155-175°C, for example, the crystallization temperature may be 155°C, 160°C, 165°C, 170°C, 175°C, or in a range between any two of the above values; crystallization time: 2-8 d, for example, the crystallization time may be 2 d, 3 d, 4 d, 5 d, 6 d, 7 d, 8 d, or in a range between any two of the above values.

In some embodiments, step S1 further comprises washing and drying the mixture after the crystallization and before the first calcination. In some embodiments, the washing method can be conventional choices in the art, as long as the purpose of the present disclosure can be achieved. In some embodiments, the drying conditions can be conventional choices in the art, as long as the purpose of the present disclosure can be achieved. In the present disclosure, drying in an oven at 100°C overnight is exemplified to illustrate the advantages of the present disclosure.

In some embodiments, the conditions for the first calcination and the second calcination can be conventional choices in the art, as long as the purpose of the present disclosure can be achieved. In the present disclosure, calcining in air at 550°C for 6 h is exemplified for both the first calcination and the second calcination to illustrate the advantages of the present disclosure.

In some embodiments, the method of ion exchange is a conventional technical choice in the art, as long as the purpose of the present disclosure can be achieved. In the present disclosure, ammonium exchange is exemplified to illustrate the advantages of the present disclosure.

In some embodiments, in step S2, the solvent includes an inorganic solvent such as water; a neutral organic solvent such as toluene, benzene, n-hexane, etc.

In some embodiments, in step S2, the mercapto compound is selected from one or more of mercaptoethanol, thioglycolic acid, 3-mercaptopropyltrimethoxysilane, 3-mercaptopropyltriethoxysilane, dimethylsulfoniopropionate, methyl mercaptopropionate, 1,3-dimercaptopropane, or 2,3-dimercapto-1-propanol; preferably 3-mercaptopropyltrimethoxysilane or 3-mercaptopropyltriethoxysilane.

In some embodiments, in step S2, the mass ratio of the solvent to the molecular sieve is in a range of 10-200:1,

preferably 20-100:1, for example, may be 10:1, 20:1, 30:1, 40:1, 50:1, 60:1, 70:1, 80:1, 90:1, 100:1, 110:1, 120:1, 130:1, 140:1, 150:1, 160:1, 170:1, 180:1, 190:1, 200:1, or in a range between any two of the above values. In some embodiments, the mass ratio of the mercapto compound to the hydrogen-form SCM-36 molecular sieve is in a range of 0.0001-0.5, preferably 0.0005-0.5, for example, may be 0.0001, 0.0005, 0.001, 0.005, 0.01, 0.05, 0.1, 0.2, 0.3, 0.4, 0.5, or in a range between any two of the above values.

In some embodiments, step S2 further comprises thoroughly stirring the reaction solution A before filtration, with conditions for stirring including: temperature in a range of 25-30°C, and time in a range of 5-48 h.

In some embodiments, in step S2, the method of washing under reflux is a conventional technical choice in the art, as long as the purpose of the present disclosure can be achieved. In the present disclosure, washing under reflux with dichloromethane and ethyl acetate is exemplified to illustrate the advantages of the present disclosure. In some embodiments, in step S2, the mass ratio of dichloromethane to the hydrogen-form SCM-36 molecular sieve is in a range of 10-80:1, for example, may be 10:1, 20:1, 30:1, 40:1, 50:1, 60:1, 70:1, 80:1, or in a range between any two of the above values; the mass ratio of ethyl acetate to the hydrogen-form SCM-36 molecular sieve is in a range of 10-80:1, for example, may be 10:1, 20:1, 30:1, 40:1, 50:1, 60:1, 70:1, 80:1, or in a range between any two of the above values; the time for washing under reflux is in a range of 6-30 h, for example, may be 6 h, 8 h, 10 h, 12 h, 14 h, 16 h, 18 h, 20 h, 22 h, 24 h, 26 h, 28 h, 30 h, or in a range between any two of the above values.

In some embodiments, in step S3, the type of oxidizing agent is not particularly limited and can be conventional choices in the art. Preferably, the oxidizing agent is hydrogen peroxide or oxygen.

In some embodiments, in step S3, the mass ratio of the oxidizing agent to the hydrogen-form SCM-36 molecular sieve is in a range of 1-100:1, preferably 2-60:1, for example, may be 1:1, 2:1, 3:1, 4:1, 5:1, 6:1, 7:1, 8:1, 9:1, 10:1, 20:1, 30:1, 40:1, 50:1, 60:1, 70:1, 80:1, 90:1, 100:1, or in a range between any two of the above values.

In some embodiments, in step S3, the alcohol is one or more of methanol or ethanol. The mass ratio of the alcohol to the hydrogen-form SCM-36 molecular sieve is in a range of 100-300:1, for example, may be 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, or in a range between any two of the above values.

In some embodiments, in step S3, the mass ratio of water to the hydrogen-form SCM-36 molecular sieve is in a range of 200-600:1, for example, may be 200:1, 250:1, 300:1, 350:1, 400:1, 450:1, 500:1, 550:1, 600:1, or in a range between any two of the above values.

In some embodiments, in step S3, mixing the solid B, oxidizing agent, alcohol, and water uniformly is achieved by stirring at room temperature, with conditions for stirring at room temperature including: temperature in a range of 25-30°C, and time in a range of 8-30 h.

In some embodiments, in step S3, the post-treatment after filtration includes adding dilute acid, stirring, filtering, washing, and drying. In some embodiments, the dilute acid is at least one of dilute hydrochloric acid, dilute sulfuric acid, and dilute nitric acid, preferably dilute sulfuric acid. In some embodiments, the concentration of the dilute acid is in a range of 0.1-1.0 mol/L, and the amount of dilute acid added is in a range of 10-100 mL per 1 g of molecular sieve; the conditions for stirring in the dilute acid include: temperature in a range of 25-30°C, and time in a range of 5-48 h. The washing method can be conventional choices in the art, as long as the purpose of the present disclosure can be achieved, for example, washing can be performed with water, alcohol, or a mixture thereof. In some embodiments, the drying conditions can be conventional choices in the art, as long as the purpose of the present disclosure can be achieved.

Hydrogenolysis Step (2)

According to the present disclosure, in step (2), the reaction product liquid containing HMF and RMF obtained from the dehydration step (1) and hydrogen is subjected to a hydrogenolysis reaction in the presence of a catalyst, undergoing the cleavage of carbon-oxygen bonds while the protecting groups are removed, to generate 2,5-dimethylfuran. By employing the preparation method according to the present disclosure, the reaction product liquid obtained by filtrating the reaction mixture produced in the dehydration step (1) to remove the solid acid catalyst, can be directly used in the hydrogenolysis step (2) without any complex intermediate product separation or solvent replacement steps, greatly simplifying the process. At the same time, by controlling the molar ratio of HMF to RMF in the reaction product liquid used as the feedstock for step (2) within a preferable range, high selectivity and high yield of the target product are ensured.

In some embodiments, in step (2), the mass ratio of the reaction product liquid containing 5-hydroxymethylfurfural and 5-(C2-C5 alkoxymethyl)furfural obtained in step (1) to the hydrogenolysis catalyst is in a range of 30-200:1, preferably 50-150:1, for example, may be 30:1, 40:1, 50:1, 60:1, 70:1, 80:1, 90:1, 100:1, 110:1, 120:1, 130:1, 140:1, 150:1, 160:1, 170:1, 180:1, 190:1, 85:1, 90:1, 95:1, 100:1, or in a range between any two of the above values.

In some embodiments, the reaction in step (2) is carried out under a hydrogen atmosphere. The hydrogen pressure is in a range of 0.5-4.0 MPa, preferably 1-3.0 MPa, for example, may be 0.5 MPa, 0.6 MPa, 0.7 MPa, 0.8 MPa, 0.9 MPa, 1 MPa, 1.5 MPa, 2 MPa, 2.5 MPa, 3.0 MPa, 3.5 MPa, 4.0 MPa, or in a range between any two of the above values.

In some embodiments, in step (2), the reaction temperature is in a range of 130-220°C, preferably 150-200°C,

more preferably 170-200°C, for example, may be 130°C, 140°C, 150°C, 160°C, 170°C, 180°C, 190°C, 200°C, or in a range between any two of the above values; the reaction time is in a range of 1-12 h, preferably 2-10 h, for example, may be 1 h, 2 h, 3 h, 4 h, 5 h, 6 h, 7 h, 8 h, 9 h, 10 h, 11 h, 12 h, or in a range between any two of the above values.

In some embodiments, the product obtained in step (2) can be separated and/or purified by conventional methods to obtain 2,5-dimethylfuran, such as distillation separation, etc.

In some embodiments, in step (2), the hydrogenolysis catalyst is a supported noble metal and/or non-noble metal catalyst, preferably selected from one or more of Pd/C, Pt/C, Ru/C, $Pd/Al_2O_3$, $Pt/Al_2O_3$, $Ru/Al_2O_3$, $Ru/Co_3O_4$, $Ni-Co_3O_4/C$, $Ni/Al_2O_3$, Ni-Fe/C, $Ni-C_3N_4/HC$ catalyst and Ni-SCM-X molecular sieve catalyst; more preferably selected from Ni-SCM-X molecular sieve catalyst and $Ni-C_3N_4/HC$ catalyst.

Ni-SCM-X Molecular Sieve Catalyst

The Ni-SCM-X molecular sieve catalyst is a solid catalyst with Ni supported on an SCM-X molecular sieve. In some embodiments, X in the SCM-X and Ni-SCM-X is 14 or 15; that is, the SCM-X molecular sieve is an SCM-14 molecular sieve, an SCM-15 molecular sieve, or a combination of an SCM-14 molecular sieve and an SCM-15 molecular sieve. The SCM-14 molecular sieve and its preparation method are described in CN109081360A and WO2018227849A1, the entire contents of which are incorporated herein by reference. The SCM-15 molecular sieve and its preparation method are described in CN109081359A and WO2018227850A1, the entire contents of which are incorporated herein by reference.

In some embodiments, in the Ni-SCM-X molecular sieve catalyst, the Si/Ge molar ratio of the SCM-X molecular sieve (after calcination) is in a range of 1.5-8, preferably 2-7. For example, the Si/Ge molar ratio may be 2, 3, 4, 5, 6, or 7, or in a range between any two of the above values. In the present disclosure, inductively coupled plasma atomic emission spectrometry can be used to measure the content of metal elements. The sample can be dissolved with hydrofluoric acid and then tested to obtain the content of metal elements.

In some embodiments, in the Ni-SCM-X molecular sieve catalyst, Ni comprises metallic Ni and $Ni^{2+}$, wherein metallic Ni means that Ni exists in metallic (zero-valent) form, wherein the $Ni/Ni^{2+}$ molar ratio is in a range of 0.02-0.15, preferably 0.05-0.12. For example, the $Ni/Ni^{2+}$ molar ratio may be 0.05, 0.06, 0.07, 0.08, 0.09, 0.10, 0.11, 0.12, 0.13, or in a range between any two of the above values.

In some embodiments, in the Ni-SCM-X molecular sieve catalyst, based on the mass of the SCM-X molecular sieve, the content of Ni, calculated as elemental Ni, is in a range of 0.2 wt%-5.0 wt%, preferably 0.5 wt%-3.0 wt%. For example, based on the mass of the SCM-X molecular sieve, the content of Ni, calculated as elemental Ni, may be 0.5 wt%, 0.6 wt%, 0.7 wt%, 0.8 wt%, 0.9 wt%, 1 wt%, 1.5 wt%, 2 wt%, 2.5 wt%, 3 wt%, or in a range between any two of the above values.

In some embodiments, in the Ni-SCM-X molecular sieve catalyst, Ni is present as nickel particles. In some embodiments, the mean particle size of the nickel particles in the Ni-SCM-X molecular sieve catalyst is in a range of 2.0-8.0 nm, preferably 3.0-7.0 nm, preferably 3.0-6.5 nm, more preferably 3.0-6.0 nm.

In some embodiments, the total specific surface area of the Ni-SCM-X molecular sieve catalyst is in a range of 90-370 $m^2 \cdot g^{-1}$, preferably 100-350 $m^2 \cdot g^{-1}$. In some embodiments, the total specific surface area of the Ni-SCM-X molecular sieve catalyst may be 100 $m^2 \cdot g^{-1}$, 110 $m^2 \cdot g^{-1}$, 120 $m^2 \cdot g^{-1}$, 130 $m^2 \cdot g^{-1}$, 140 $m^2 \cdot g^{-1}$, 150 $m^2 \cdot g^{-1}$, 160 $m^2 \cdot g^{-1}$, 200 $m^2 \cdot g^{-1}$, 250 $m^2 \cdot g^{-1}$, 300 $m^2 \cdot g^{-1}$, or in a range between any two of the above values. In the present disclosure, the specific surface area is measured by the BET method.

In some embodiments, the total acid amount of the Ni-SCM-X molecular sieve catalyst is in a range of 130-220 $\mu mol \cdot g^{-1}$, preferably 150-200 $\mu mol \cdot g^{-1}$. In some embodiments, the total acid amount of the Ni-SCM-X molecular sieve catalyst is 150 $\mu mol \cdot g^{-1}$, 160 $\mu mol \cdot g^{-1}$, 170 $\mu mol \cdot g^{-1}$, 180 $\mu mol \cdot g^{-1}$, 190 $\mu mol \cdot g^{-1}$, 200 $\mu mol \cdot g^{-1}$, or in a range between any two of the above values. In the present disclosure, the total acid amount is the total acid amount determined by $NH_3$ temperature-programmed desorption ($NH_3$-TPD) experiment.

In some embodiments, the Brønsted acid/Lewis acid ratio of the Ni-SCM-X molecular sieve catalyst is in a range of 0.3-0.8, preferably 0.35-0.6, more preferably 0.4-0.6. In the present disclosure, the acid type and acid amount are determined by pyridine adsorption infrared spectroscopy, and the Brønsted acid/Lewis acid ratio is calculated.

In some embodiments, the method for preparing the Ni-SCM-X molecular sieve catalyst comprises:
mixing an SCM-X molecular sieve with a nickel-containing precursor to obtain a mixture, and calcining and reducing the obtained mixture to obtain the Ni-SCM-X molecular sieve catalyst, wherein X is 14 or 15.

In some embodiments, in the method for preparing the Ni-SCM-X molecular sieve catalyst, before calcining and reducing the mixture, the mixture is subjected to drying.

In some embodiments, the nickel-containing precursor can be selected from nickel precursors containing organic ligand(s) (organometallic precursors). In some embodiments, preferably, the organic ligand is selected from organic ligands containing a five-membered carbocyclic ring, a six-membered carbocyclic ring, or an eight-membered carbocyclic ring. In some embodiments, preferably the five-membered carbocyclic ring may be a cyclopentadiene ring, the six-membered carbocyclic ring may be a benzene ring, and the eight-membered carbocyclic ring may be a cyclooctadiene

ring. The five-membered carbocyclic ring, six-membered carbocyclic ring or eight-membered carbocyclic ring optionally have substituent(s), for example C1-C4 alkyl group. In some embodiments, preferably the nickel-containing precursor is selected from at least one of nickelocene (bis(cyclopentadienyl) nickel), bis-(1,5-cyclooctadiene) nickel, bis(tetramethyl-cyclopentadienyl) nickel, 1,2-bis(diphenylphosphino)ethane nickel chloride and bis(triphenylphosphine) nickel chloride, and further more preferably nickelocene.

In some embodiments, in the mixing step, the mass ratio of Ni (the amount of Ni in the nickel-containing precursor) to the SCM-X molecular sieve is in a range of 0.2-8.0:100, preferably 0.5-5.0:100. In some embodiments, for example, the mass ratio of Ni (the amount of Ni in the nickel-containing precursor) to the SCM-X molecular sieve may be 0.6:100, 0.7:100, 0.8:100, 0.9:100, 1:100, 1.5:100, 2:100, 2.5:100, 3:100, 3.5:100, 4:100, 4.5:100, or in a range between any two of the above values.

In some embodiments, the mixing is physical mixing. The mixing includes but is not limited to grinding, ball milling, etc. For example, the grinding can be carried out in a mortar. For example, the ball milling can be carried out in a ball mill. A person skilled in the art can appropriately determine the mixing time as long as uniform mixing can be achieved.

In some embodiments, after mixing the SCM-X molecular sieve with the nickel-containing precursor, a uniform mixture is obtained. After mixing, optionally, the obtained mixture can be dried. The drying temperature may be in a range of 50-130°C, for example, may be 60°C, 70°C, 80°C, 90°C, 100°C, 110°C, 120°C, 130°C, or in a range between any two of the above values. The drying time may be in a range of 2-8 h, for example, may be 3 h, 4 h, 5 h, 6 h, 7 h, 8 h, or in a range between any two of the above values. In some embodiments, the conditions for drying can include drying at 50-130°C for 2-8 h. Drying may be carried out, for example, in an air atmosphere.

In some embodiments, after optional drying, the dried product is calcined. The calcination temperature may be in a range of 300-650°C, for example, may be 350°C, 400°C, 450°C, 500°C, 550°C, 600°C, 620°C, 650°C, or in a range between any two of the above values. The calcination time may be in a range of 1-12 h, for example, may be 2 h, 3 h, 4 h, 5 h, 6 h, 7 h, 8 h, 9 h, 10 h, 11 h, 12 h, or in a range between any two of the above values. The calcination atmosphere may be an oxygen-containing gas, preferably oxygen, air, or a mixed gas of oxygen and air. In some embodiments, the conditions for calcining can include: calcining at 300-650°C for 1-12 h, and the calcination atmosphere is an oxygen-containing gas, preferably oxygen, air, or a mixed gas of oxygen and air. When a mixed gas of oxygen and air is used, oxygen and air may be mixed in any proportion.

In some embodiments, after calcining, the calcined product is subjected to a reduction treatment. In some embodiments, the reduction is carried out under a hydrogen atmosphere. The reduction temperature may be in a range of 300-500°C, preferably 350-450°C, for example, may be 360°C, 370°C, 380°C, 390°C, 400°C, 410°C, 420°C, 430°C, 440°C, 450°C, or in a range between any two of the above values. The reduction time may be in a range of 1-8 h, for example, may be 2 h, 3 h, 4 h, 5 h, 6 h, 7 h, 8 h, or in a range between any two of the above values. In some embodiments, the reduction temperature may be in a range of 300-500°C, preferably 350-450°C; and the reduction time may be in a range of 1-8 h, preferably 2-6 h. In some embodiments, the reduction is carried out under a hydrogen atmosphere at atmospheric pressure.

## Ni-$C_3N_4$/HC Catalyst

An exemplary Ni-$C_3N_4$/HC catalyst and its preparation method are described in the literature: Chinese Journal of Catalysis [J]. 2024, 60, 253-261, the entire contents of which are incorporated herein by reference.

In some embodiments, the Ni-$C_3N_4$/HC catalyst comprises nickel, $C_3N_4$, and a support, wherein the support is activated carbon activated with hydrogen (HC).

In some embodiments, the Ni-$C_3N_4$/HC catalyst comprises Ni particles encapsulated by graphitic $C_3N_4$ shell. In some embodiments, the mean particle size of the Ni particles is in a range of 1.2-6.0 nm, preferably 1.2-5.0 nm, more preferably 1.7-3.5 nm.

In some embodiments, in the Ni-$C_3N_4$/HC catalyst, based on the mass of the support, the content of nickel, calculated as elemental Ni, is in a range of 0.5 wt%-10 wt%, and the content of $C_3N_4$ is in a range of 2 wt%-30 wt%; preferably, the content of nickel, calculated as elemental Ni, is in a range of 1 wt%-6 wt%, and the content of $C_3N_4$ is in a range of 4 wt%-20 wt%. In some embodiments, based on the mass of the support, the content of nickel, calculated as elemental Ni, may be 0.5 wt%, 0.6 wt%, 0.7 wt%, 0.8 wt%, 0.9 wt%, 1 wt%, 2 wt%, 3 wt%, 4 wt%, 5 wt%, 6 wt%, 7 wt%, 8 wt%, 9 wt%, 10 wt%, or in a range between any two of the above values, and the content of $C_3N_4$ may be 2 wt%, 3 wt%, 4 wt%, 5 wt%, 6 wt%, 7 wt%, 8 wt%, 9 wt%, 10 wt%, 12 wt%, 15 wt%, 18 wt%, 20 wt%, 22 wt%, 25 wt%, 28 wt%, 30 wt%, or in a range between any two of the above values.

In some embodiments, the oxygen content of the activated carbon activated with hydrogen (HC) is in a range of 4 wt%-18 wt%, preferably 6 wt%-15 wt%, for example, may be 4 wt%, 5 wt%, 6 wt%, 7 wt%, 8 wt%, 9 wt%, 10 wt%, 11 wt%, 12 wt%, 13 wt%, 14 wt%, 15 wt%, 16 wt%, 17 wt%, 18 wt%, or in a range between any two of the above values.

In some embodiments, the total specific surface area of the Ni-$C_3N_4$/HC catalyst is in a range of 700-1800 $m^2 \cdot g^{-1}$, preferably 800-1300 $m^2 \cdot g^{-1}$. In some embodiments, the total specific surface area of the Ni-$C_3N_4$/HC catalyst may be 700

$m^2 \cdot g^{-1}$, 800 $m^2 \cdot g^{-1}$, 900 $m^2 \cdot g^{-1}$, 1000 $m^2 \cdot g^{-1}$, 1100 $m^2 \cdot g^{-1}$, 1200 $m^2 \cdot g^{-1}$, 1300 $m^2 \cdot g^{-1}$, 1400 $m^2 \cdot g^{-1}$, 1500 $m^2 \cdot g^{-1}$, 1600 $m^2 \cdot g^{-1}$, 1700 $m^2 \cdot g^{-1}$, 1800 $m^2 \cdot g^{-1}$, or in a range between any two of the above values. In the present disclosure, the specific surface area is measured by the BET method.

In some embodiments, the molar ratio of the $Ni_3N$ species to the oxidized NiO in the Ni-$C_3N_4$/HC catalyst is in a range of 0.25-0.60:1, preferably 0.30-0.50:1, for example, may be 0.25:1, 0.30:1, 0.35:1, 0.40:1, 0.45:1, 0.50:1, 0.55:1, 0.60:1, or in a range between any two of the above values.

In some embodiments, the method for preparing the Ni-$C_3N_4$/HC catalyst comprises:

subjecting activated carbon to activation under a hydrogen atmosphere to obtain activated carbon activated with hydrogen (HC);

loading a nickel source and a nitrogen-containing precursor onto the activated carbon activated with hydrogen, followed by drying, calcining, and reducing to obtain the Ni-$C_3N_4$/HC catalyst.

In some embodiments, the method comprises first preparing a solution, preferably an aqueous solution, of the nickel source and the nitrogen-containing precursor, then mixing the solution with the activated carbon activated with hydrogen, followed by drying, calcining, and reducing to obtain the catalyst.

In some embodiments, the mass ratio of the nickel source, the nitrogen-containing precursor, the activated carbon activated with hydrogen, and the solvent (preferably water) is in a range of 0.004-0.2 : 0.02-0.40 : 1 : 1-10, preferably 0.008-0.1 : 0.05-0.30 : 1 : 2-8, wherein the nickel source is calculated based on elemental nickel, and the nitrogen-containing precursor is calculated based on the total mass of elements C and N.

In some embodiments, the hydrogen activation temperature is in a range of 500-1200°C, preferably 600-1000°C, for example, may be 500°C, 600°C, 700°C, 800°C, 900°C, 1000°C, 1100°C, 1200°C, or in a range between any two of the above values; the activation time is in a range of 2-12 h, preferably 3-10 h, for example, may be 2 h, 3 h, 4 h, 5 h, 6 h, 7 h, 8 h, 9 h, 10 h, 11 h, 12 h, or in a range between any two of the above values; and the activation atmosphere is hydrogen or a mixed gas of hydrogen and argon.

In some embodiments, the drying temperature is in a range of 50-120°C, for example, may be 50°C, 60°C, 70°C, 80°C, 90°C, 100°C, 110°C, 120°C, or in a range between any two of the above values, and the drying time is in a range of 4-12 h, for example, may be 4 h, 5 h, 6 h, 7 h, 8 h, 9 h, 10 h, 11 h, 12 h, or in a range between any two of the above values.

In some embodiments, the calcination temperature is in a range of 300-650°C, preferably 350-600°C, for example, may be 300°C, 350°C, 400°C, 450°C, 500°C, 550°C, 600°C, or in a range between any two of the above values; the calcination time is in a range of 1-12 h, preferably 2-6 h, for example, may be 1 hour, 2 h, 3 h, 4 h, 5 h, 6 h, 7 h, 8 h, 9 h, 10 h, 11 h, 12 h, or in a range between any two of the above values; and the calcination atmosphere is a non-oxygen gas atmosphere; preferably, the non-oxygen gas includes at least one of nitrogen and argon. When a mixed gas of nitrogen and argon is used, nitrogen and argon may be mixed in any proportion.

In some embodiments, the reduction temperature is in a range of 300-700°C, preferably 350-550°C, for example, may be 300°C, 350°C, 400°C, 450°C, 500°C, 550°C, 600°C, 650°C, 700°C, or in a range between any two of the above values; the reduction time is in a range of 1-12 h, preferably 2-6 h, for example, may be 1 hour, 2 h, 3 h, 4 h, 5 h, 6 h, 7 h, 8 h, 9 h, 10 h, 11 h, 12 h, or in a range between any two of the above values; and the reduction atmosphere is hydrogen or a mixed gas of hydrogen and argon.

In some embodiments, the nickel source is selected from soluble nickel salts, preferably selected from at least one of nickel nitrate, nickel acetate, nickel chloride, nickel acetylacetonate, nickel sulfate, and combinations thereof, more preferably selected from at least one of nickel nitrate, nickel acetate, and combinations thereof.

In some embodiments, the nitrogen-containing precursor is selected from organic nitrogen-containing compounds, preferably selected from at least one of urea, cyanoguanidine, melamine, and combinations thereof, more preferably selected from at least one of urea, cyanoguanidine, and combinations thereof.

In some embodiments, the activated carbon is selected from at least one of coconut shell activated carbon, and wood-based activated carbon, preferably coconut shell activated carbon. In some embodiments, the oxygen content of the coconut shell activated carbon is in a range of 20 wt%-40 wt%, preferably 25 wt%-38 wt%, for example, may be 20 wt%, 22 wt%, 25 wt%, 28 wt%, 30 wt%, 32 wt%, 35 wt%, 38 wt%, 40 wt%, or in a range between any two of the above values.

In the present disclosure, pressure refers to gauge pressure, unless otherwise specified.

In the present disclosure, room temperature refers to a temperature of about 25°C, unless otherwise specified.

In the present disclosure, the XRD measurement method included: using a Rigaku (Japan) Ultima IV X-ray powder diffractometer, CuK$\alpha$ radiation source ($\lambda$ = 1.54 Å), nickel filter, 2$\theta$ scanning range 2°-50°/75°, operating voltage 35 kV, current 25 mA, scanning rate 10°/min.

In the present disclosure, the model of the inductively coupled plasma atomic emission spectrometer (ICP) used was Varian 725-ES. The sample was dissolved with hydrofluoric acid to detect the content of elements.

In the present disclosure, $NH_3$ temperature-programmed desorption ($NH_3$-TPD) experiments were carried out on a TPD/TPR Altamira AMI-3300 instrument. The obtained spectra were fitted and peak-separated to calculate the total

acid amount.

In the present disclosure, the acid amount and acid type of the catalyst were determined by pyridine adsorption infrared spectroscopy. The instrument used was a Nicolet Model 710 spectrometer. The specific operation steps were as follows: a. Sample pretreatment: the sample (about 30 mg) was pressed into a thin circular disk with a diameter of 13 mm and placed in an infrared sample cell; then, the sample was pretreated at 400°C for 1 h under vacuum cell conditions; after the sample cell was cooled to room temperature, the infrared data of the sample was scanned as background; b. Pyridine adsorption: at room temperature and under vacuum environment, pyridine vapor was introduced into the in-situ cell until adsorption reached equilibrium, the adsorption time was 1 h, and then the infrared absorption spectrum was scanned and recorded; c. Pyridine desorption: after adsorption, vacuum was applied at 100°C until the internal pressure no longer changed (about $10^{-3}$ Pa), the desorption time was 40 min, and then the infrared absorption spectrum was scanned and recorded. The difference spectrum before and after pyridine adsorption was the obtained pyridine adsorption-infrared absorption spectrum. According to the spectrum, the acid amount of the sample was calculated using the following formulas:

$$C_L = K_L \times A_{1440} = \frac{\pi}{IMEC_L} \times \left(\frac{r^2}{w}\right) \times A_{1440}$$

$$C_B = K_B \times A_{1540} = \frac{\pi}{IMEC_B} \times \left(\frac{r^2}{w}\right) \times A_{1540}$$

wherein, r and w are the diameter (cm) and mass (g) of the thin circular disk of the catalyst, respectively, and A is the integrated absorbance value of the peak at the specified wavenumber according to the scanned pyridine adsorption-infrared absorption spectrum. IMEC is the integrated molar extinction coefficient, wherein $IMEC_L$ is 2.22 and $IMEC_B$ is 1.67, $C_L$ is the Lewis acid amount ($\mu mol \cdot g^{-1}$), and $C_B$ is the Brønsted acid amount ($\mu mol \cdot g^{-1}$).

In the present disclosure, the model of the scanning electron microscope (SEM) used was Hitachi S-4800II field emission scanning electron microscope.

In the present disclosure, the model of the transmission electron microscope (TEM) used was JEOL JEM-2100F electron microscope (200 kV). The mean particle size of metal particles (such as nickel particles) in the catalyst was obtained by randomly selecting about 30 metal particles (such as nickel particles) from the TEM image for particle size statistics, and then calculating the arithmetic mean value.

In the present disclosure, the determination of the surface element binding energy of the catalyst was carried out on a Thermo ESCA LAB-250 X-ray photoelectron spectrometer, using C1s = 284.6 eV as an internal standard to calibrate the measured element signals.

In the present disclosure, the specific surface area and external specific surface area of the catalyst were measured by the nitrogen physical adsorption-desorption method (BET method). The nitrogen physical adsorption-desorption isotherms of the molecular sieve were measured using a physical adsorption instrument (Micromeritics ASAP 2020M). The obtained results were analyzed by the Brunauer-Emmet-Teller (BET) method to obtain the specific surface area of the sample, and by the Barrett-Joyner-Halenda (BJH) method to obtain the pore volume and pore size distribution of the sample. Experimental conditions: the sample was vacuum degassed at 350°C for 4 h before analysis to remove moisture and volatile impurities, and then the nitrogen adsorption-desorption test was performed in a liquid nitrogen environment (-196°C).

In the present disclosure, 5-hydroxymethylfurfural (HMF), 5-(C2-C5 alkoxymethyl)furfural (RMF), and 2,5-dimethylfuran (DMF) were qualitatively analyzed by gas chromatography-mass spectrometry (GC-MS). The conversion of hexose, the yield and conversion of intermediate products HMF and RMF, and the yield of the reaction product DMF were analyzed by gas chromatography (GC) and high-performance liquid chromatography (HPLC). The gas chromatography-mass spectrometer was an Agilent (USA) G7077BA\MSD, with an INNOWAX gas chromatography capillary column (60 m, 0.32 mm); the gas chromatograph was an Agilent 8860, with a flame ionization detector (FID), and an INNOWAX gas chromatography capillary column (60 m, 0.32 mm). The high-performance liquid chromatograph model was Agilent 1260 Prime II, and signal detection was performed by a refractive index detector (RID). The chromatographic column used included a Hi-Plex H column, with dilute sulfuric acid as the mobile phase, a flow rate of 0.6 mL/min, and a column temperature of 60°C.

The process for preparing DMF from a biomass feedstock include two steps. The first step involves a dehydration reaction of the biomass feedstock to prepare HMF and RMF, wherein the calculation formula for the yield of HMF and RMF is:

Yield of HMF and RMF % = (moles of HMF and RMF produced in the first step reaction, $n_1$) / (moles of hexose units in the reaction substrate biomass, $n_0$) × 100%; wherein, the chemical formula of hexose is $C_6H_{12}O_6$.

In the second step, the HMF and RMF ($n_1$) produced in the first step reaction were used as a feedstock, which is subjected to a hydrogenolysis reaction with hydrogen to prepare DMF, wherein the calculation formula for the conversion of HMF and RMF is:

Conversion of HMF and RMF % = (moles of HMF and RMF remaining after the second step reaction, $n_2$) / (moles of HMF and RMF in the reactants of the second step reaction, $n_1$) × 100%.

In the second step, the formula for the selectivity of the product DMF is:

Selectivity of DMF % = (moles of DMF produced by the reaction, $n_3$) / (moles of HMF and RMF in the reactants of the second step reaction, $n_1$ - moles of HMF and RMF remaining after the second step reaction, $n_2$) × 100%.

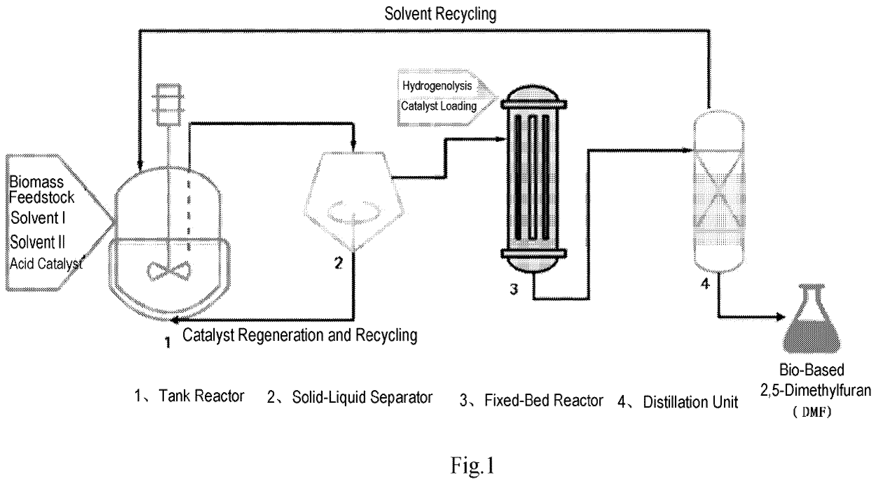

The reaction equipment and process for preparing 2,5-dimethylfuran by biomass conversion according to the present disclosure are described below with reference to FIG. 1:

(1) The biomass feedstock, solvent I, solvent II, and solid acid catalyst are charged into a tank reactor 1, and reacted under an inert atmosphere with stirring to produce a reaction product stream containing 5-hydroxymethylfurfural and 5-(C2-C5 alkoxymethyl)furfural; the reaction product stream is sent to a solid-liquid separator 2 for solid-liquid separation to obtain a reaction product liquid containing 5-hydroxymethylfurfural and 5-(C2-C5 alkoxymethyl)furfural and the solid acid catalyst, and the obtained solid acid catalyst is regenerated and recycled to the tank reactor 1;
(2) The reaction product liquid obtained in step (1) is sent to a fixed-bed reactor 3 loaded with a hydrogenolysis catalyst, where the 5-hydroxymethylfurfural and 5-(C2-C5 alkoxymethyl)furfural contained in the reaction product liquid react with hydrogen to produce a reaction product containing 2,5-dimethylfuran; the obtained reaction product containing 2,5-dimethylfuran is sent to a distillation unit 4 for subsequent separation to obtain 2,5-dimethylfuran.

To facilitate understanding of the present disclosure, the present disclosure provides examples as follows. However, these examples are only for helping to understand the present disclosure and should not be considered as specific limitations on the present disclosure.

**Examples**

Unless otherwise specified, the experimental methods used in the following examples and comparative examples were conventional methods. Unless otherwise specified, the materials, reagents, etc., used in the following examples and comparative examples were commercially available, with a purity of analytical grade.

**Preparation Example 1**

14.35 g of deionized water, 0.604 g of sodium aluminate (containing 40.5 wt% $Al_2O_3$, 30.6 wt% $Na_2O$, balance water), 0.366 g of sodium hydroxide, 7.06 g of trimethylethylammonium hydroxide solution (containing 25.00 wt% trimethylethylammonium hydroxide, balance water) (organic structure-directing agent), and 12.60 g of silica sol (containing 40.0 wt% $SiO_2$, balance water) were mixed and stirred at room temperature for 4 h to obtain a mixture. The final material ratio (molar ratio) was:

$$SiO_2/Al_2O_3 = 35;$$

$$OH^-/SiO_2 = 0.18;$$

Trimethylethylammonium hydroxide (R)/$SiO_2$ = 0.20;

$$H_2O/SiO_2 = 18.$$

The mixture was charged into a stainless steel autoclave and crystallized by heating at 170°C with a rotation

speed of 20 rpm for 3 days. After crystallization, the mixture was filtered, washed, and dried in an oven at 100°C overnight to obtain a product, i.e., SCM-36 molecular sieve.

The obtained SCM-36 molecular sieve was calcined to remove the organic structure-directing agent, then subjected to ion exchange and calcination to obtain hydrogen-form SCM-36. The ratio of terminal silanol groups to the sum of internal silanol groups and bridging hydroxyl groups in the hydrogen-form SCM-36 molecular sieve was 0.38.

C-FER was synthesized following the method for SCM-36, but replacing trimethylethylammonium hydroxide with cyclohexylamine, keeping other conditions unchanged. The product was calcined to remove the organic structure-directing agent, then subjected to ion exchange and calcination to obtain hydrogen-form C-FER.

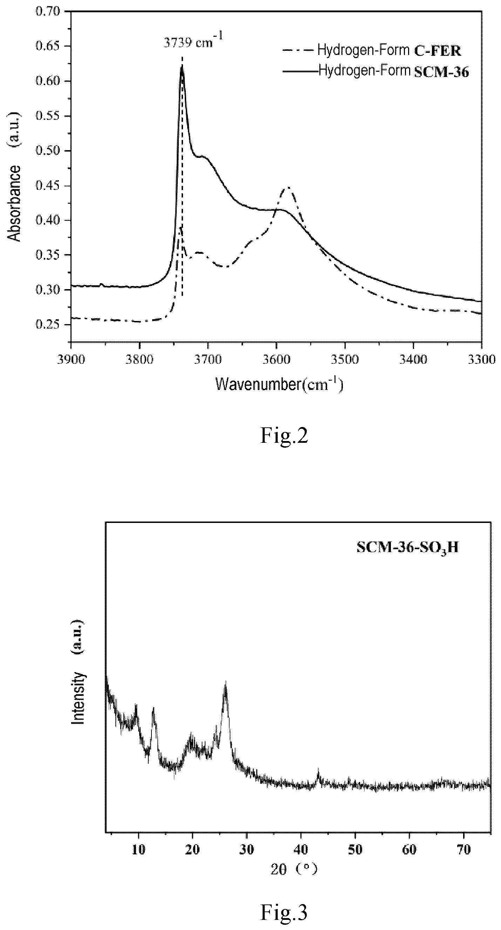

The infrared spectrum of hydrogen-form SCM-36 is shown in FIG. 2. The peak at 3739 $cm^{-1}$ is assigned to the surface silanol groups Si-OH of the molecular sieve. The figure shows that the hydrogen-form SCM-36 molecular sieve has more Si-OH than the hydrogen-form C-FER molecular sieve.

1.5 g of hydrogen-form SCM-36 molecular sieve and 1.0 g of 3-mercaptopropyltrimethoxysilane were added to 50 g of toluene and the mixture was stirred thoroughly at 25-30°C for 24 h. After stirring, the mixture was filtered, and 30.0 g of dichloromethane and 30.0 g of ethyl acetate were added to wash under reflux for 24 h. Subsequently, the obtained solid was added to a mixed solution of 40 g of $H_2O_2$ (30%), 120 g of methanol, and 400 g of deionized water, and the mixture was stirred at room temperature for 15 h. After stirring, the mixture was filtered, and the precipitate was dispersed in 40 mL of sulfuric acid (0.6 mol/L) and stirred thoroughly for 15 h. Finally, the product was thoroughly washed with ethanol and water and dried to obtain SCM-36-$SO_3H$ molecular sieve.

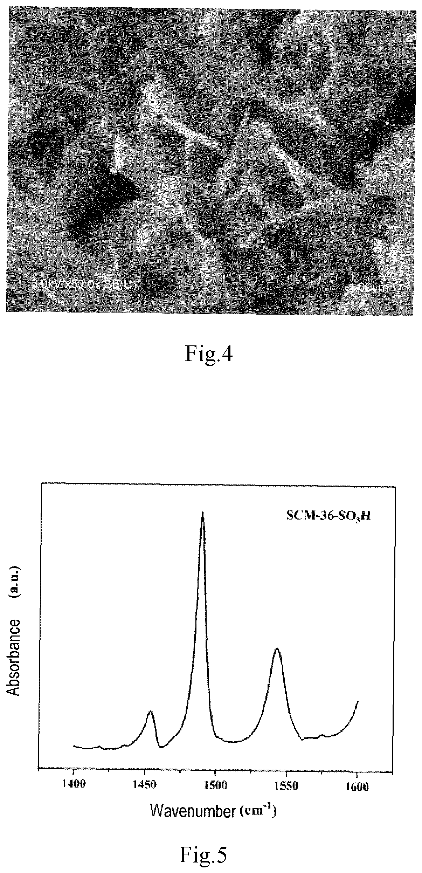

The XRD pattern of the SCM-36-$SO_3H$ molecular sieve from Preparation Example 1 is shown in FIG. 3. The SEM image from Preparation Example 1 is shown in FIG. 4. The pyridine adsorption infrared spectrum from Preparation Example 1 is shown in FIG. 5, and the Brønsted acid/Lewis acid ratio calculated therefrom was 3.3. The -$SO_3H$ content of the SCM-36-$SO_3H$ molecular sieve from Preparation Example 1, calculated from the S content determined by ICP, was 6.5 wt%, and the external specific surface area/total specific surface area ratio measured by BET was 0.55.

**Preparation Example 2**

An SCM-14 molecular sieve was synthesized according to the preparation method described in Example 1 of CN109081360A, wherein the Si/Ge molar ratio was 3.7. The nickel-containing precursor was nickelocene. The mass ratio of Ni (the amount of Ni in the organometallic precursor) to the above SCM-14 molecular sieve was 1.0:100. The amount of SCM-14 added was 3.0 g. The mixture was thoroughly ground in a mortar to obtain a uniformly dispersed mixture. The above mixture was dried in an oven at 100°C for 4 h, then calcined at 550°C for 4 h, and reduced under atmospheric hydrogen conditions at a reduction temperature of 400°C for 3 h, to obtain a Ni-SCM-14 molecular sieve.

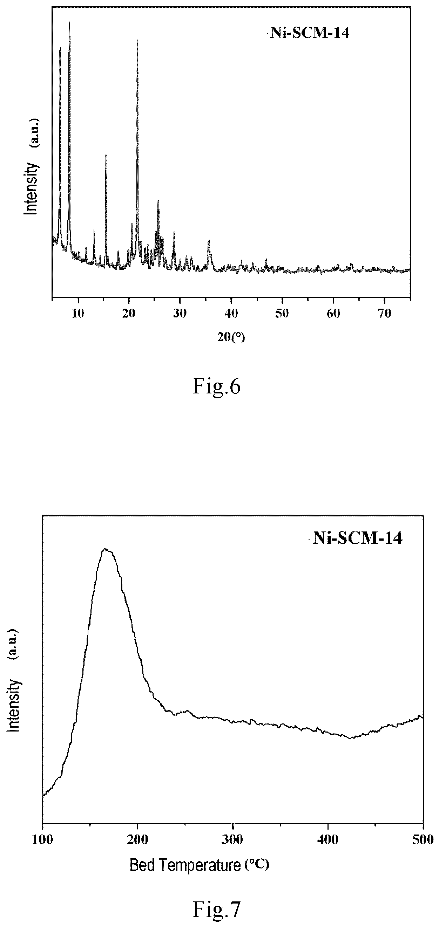

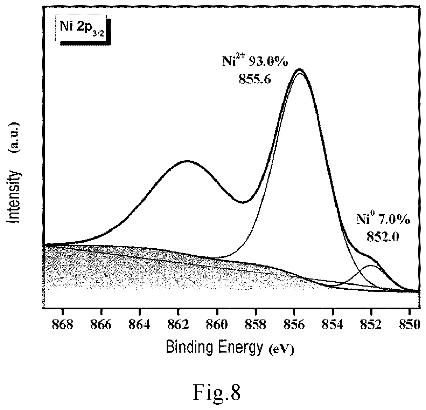

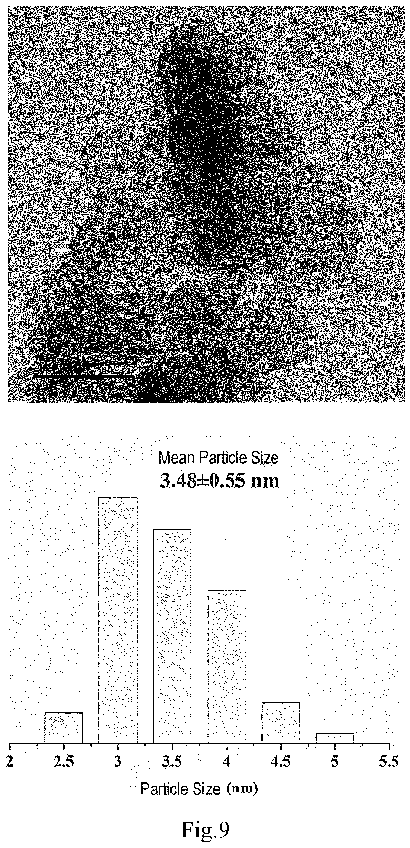

The Si/Ge molar ratio in Preparation Example 2 measured by ICP was 3.7, and the relative content of Ni was 1.0 wt%. The specific surface area in Preparation Example 2 measured by the BET method was 138 $m^2/g$. The XRD pattern of Preparation Example 2 is shown in FIG. 6. The $NH_3$-TPD pattern of Preparation Example 2 is shown in FIG. 7, and the total acid amount calculated therefrom was 175 $\mu mol \cdot g^{-1}$. The Brønsted acid/Lewis acid ratio calculated from the pyridine adsorption infrared test was 0.50. The XPS spectrum of Preparation Example 2 is shown in FIG. 8. The peak at a binding energy of 852.0 eV corresponds to metallic Ni ($2p_{3/2}$ peak), and the peak at a binding energy of 855.6 eV corresponds to divalent nickel ($2^+$). The molar ratio of metallic Ni to $Ni^{2+}$ ($Ni/Ni^{2+}$) calculated from the ratio of the two peak areas was 0.08. The TEM image and particle size distribution of Preparation Example 2 are shown in FIG. 9, showing that the distribution of nickel particles was uniform without obvious aggregation, and the mean particle size of nickel particles was 3.5 nm.

**Preparation Example 3**

An SCM-15 molecular sieve was synthesized according to the preparation method described in Example 1 of CN109081359A, wherein the Si/Ge molar ratio was 5.1. The nickel-containing precursor was nickelocene. The mass ratio of Ni (the amount of Ni in the organometallic precursor) to the above SCM-15 molecular sieve was 1.0:100. The amount of SCM-15 added was 3.0 g. The mixture was thoroughly ground in a mortar to obtain a uniformly dispersed mixture. The above product was dried in an oven at 80°C for 5 h, then calcined at 550°C for 4 h, and reduced under hydrogen conditions at a reduction temperature of 400°C for 2 h, to obtain a Ni-SCM-15 molecular sieve.

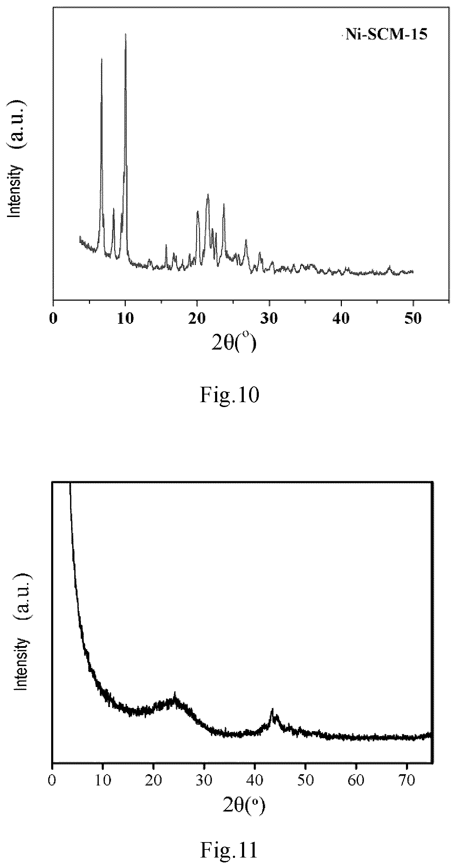

The Si/Ge molar ratio in Preparation Example 3 measured by ICP was 5.1, and the relative content of Ni was 1.0 wt%. The specific surface area of Preparation Example 3 measured by the BET method was 325 $m^2/g$. The XRD pattern of Preparation Example 3 is shown in FIG. 10. The $NH_3$-TPD of Preparation Example 3 was similar to FIG. 7, and the total acid amount calculated therefrom was 157 $\mu mol \cdot g^{-1}$. The Brønsted acid/Lewis acid ratio calculated from the pyridine adsorption infrared test was 0.46. The XPS of Preparation Example 3 was similar to FIG. 8. The peak at a binding energy of 852.0 eV corresponds to metallic Ni ($2p_{3/2}$ peak), and the peak at a binding energy of 855.6 eV corresponds to divalent nickel ($2^+$). The molar ratio of metallic Ni to $Ni^{2+}$ ($Ni/Ni^{2+}$) calculated from the ratio of the two peak areas was 0.10. The TEM of Preparation Example 3 was similar to FIG. 9, showing that the distribution of nickel was uniform without

obvious aggregation, and the mean particle size of nickel particles was 4.1 nm.

**Preparation Example 4**

Coconut shell activated carbon (specific surface area: 1350 $m^2 \cdot g^{-1}$, oxygen content: 32 wt%) was activated under a hydrogen atmosphere at 650°C for 4 h to obtain activated carbon activated with hydrogen HC. The specific surface area of HC was 1280 $m^2 \cdot g^{-1}$, and the oxygen content was 11 wt%.

Under stirring conditions, 0.03 g of nickel nitrate and 0.22 g of urea were dissolved in 6 mL of an aqueous solution, and 1 g of activated carbon activated with hydrogen HC was added for impregnation, wherein the mass ratio of nickel nitrate (calculated as elemental nickel), urea (calculated as the total mass of elements C and N), activated carbon activated with hydrogen HC, and water was 0.009:0.15:1:6. The impregnated product was dried at 100°C for 6 h, calcined under a nitrogen atmosphere at 500°C for 4 h, and reduced under a hydrogen atmosphere at 450°C for 4 h to obtain a Ni-$C_3N_4$/HC catalyst, wherein the relative content of Ni was 1 wt%, and the relative content of $C_3N_4$ was 11 wt%.

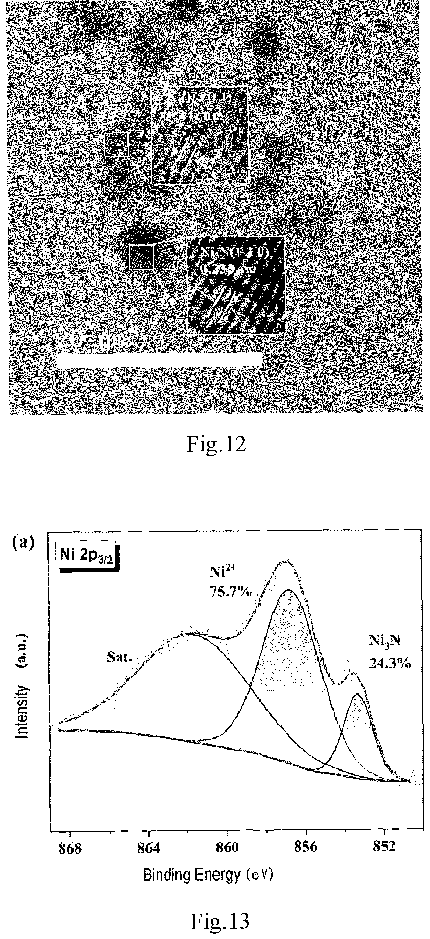

The XRD pattern of Preparation Example 4 is shown in FIG. 11. The TEM image of Preparation Example 4 is shown in FIG. 12. BET test results showed that the specific surface area of the catalyst was 1150 $m^2 \cdot g^{-1}$. The TEM image (FIG. 12) showed highly dispersed nickel nanoparticles; $Ni_3N$ and NiO phases; and graphitic $C_3N_4$ shell on the nickel particles.

In the XPS spectrum of Preparation Example 4 (FIG. 13), the peak at a binding energy of 853.3 eV corresponds to $Ni_3N$ ($2p_{3/2}$ peak), and the peak at a binding energy of 856.7 eV corresponds to divalent nickel ($2^+$). The molar ratio of $Ni_3N$ to oxidized NiO calculated from the ratio of the two peak areas was 0.32.

**Example 1**

Fructose was used as the substrate, and n-butanol (solvent I) and tetrahydrofuran (solvent II) were used as solvents. 0.5 g of the SCM-36-$SO_3H$ molecular sieve obtained in Preparation Example 1, 4.0 g of fructose (initial mass concentration: 16.7%), 10 g of tetrahydrofuran, and 10 g of n-butanol were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at a temperature of 165°C for a time of 30 min. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 2**

The test was carried out according to the method of Example 1, except that the solid acid catalyst was changed to USY-3 molecular sieve (Sinopec Catalyst Co., Ltd.). The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 3**

The test was carried out according to the method of Example 1, except that the solid acid catalyst was changed to A-15 resin (Macklin Amberlyst 15 ion exchange resin, A800632). The reaction product liquid from the first step was analyzed to obtain the fructose conversion and the yields of 5-hydroxymethylfurfural (HMF) and 5-(C2-C5 alkoxymethyl) furfural (RMF). The fructose conversion, the sum of HMF and RMF yields, and the yield ratio are shown in Table 1.

**Example 4**

The test was carried out according to the method of Example 1, except that the mass ratio of biomass feedstock to solid acid catalyst was 12, wherein 4.0 g of fructose was added (initial mass concentration: 16.7%). The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 5**

The test was carried out according to the method of Example 1, except that the mass ratio of n-butanol (solvent I) to tetrahydrofuran (solvent II) was 1.5, and the total mass of solvents was the same as in Example 1. The reaction product

liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 6**

The test was carried out according to the method of Example 1, except that the mass ratio of n-butanol (solvent I) to tetrahydrofuran (solvent II) was 2, and the total mass of solvents was the same as in Example 1. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 7**

The test was carried out according to the method of Example 1, except that the mass ratio of n-butanol (solvent I) to tetrahydrofuran (solvent II) was 0.75, and the total mass of solvents was the same as in Example 1. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 8**

The test was carried out according to the method of Example 1, except that the mass ratio of n-butanol (solvent I) to tetrahydrofuran (solvent II) was 0.5, and the total mass of solvents was the same as in Example 1. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 9**

The test was carried out according to the method of Example 1, except that the reaction temperature was 150°C. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 10**

The test was carried out according to the method of Example 1, except that the reaction temperature was 180°C. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 11**

The test was carried out according to the method of Example 1, except that the reaction time was 20 min. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 12**

The test was carried out according to the method of Example 1, except that the reaction time was 50 min. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 13**

The test was carried out according to the method of Example 1, except that the biomass feedstock was glucose. The reaction product liquid from the first step was analyzed to obtain the glucose conversion and the yields of 5-hydroxymethylfurfural (HMF) and 5-(C2-C5 alkoxymethyl)furfural (RMF). The glucose conversion, the sum of HMF and RMF yields, and the yield ratio are shown in Table 1.

**Example 14**

The test was carried out according to the method of Example 1, except that solvent I was ethanol. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 15**

The test was carried out according to the method of Example 1, except that solvent II was methyl isobutyl ketone. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Example 16**

The test was carried out according to the method of Example 1, except that solvent II was 1,4-dioxane. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

**Comparative Example 1 (CE.1)**

The test was carried out according to the method of Example 1, except that solvent I and solvent II were replaced with an equal amount (20 g) of water. The reaction product liquid from the first step was analyzed to obtain the fructose conversion and the target product yield; only 5-hydroxymethylfurfural (HMF) was produced. The fructose conversion and HMF yield are shown in Table 1.

**Comparative Example 2 (CE.2)**

The test was carried out according to the method of Example 1, except that solvent II was replaced with water. The reaction product liquid from the first step was analyzed to obtain the fructose conversion and the yields of the target products 5-hydroxymethylfurfural (HMF) and 5-(C2-C5 alkoxymethyl)furfural (RMF). The fructose conversion, the sum of HMF and RMF yields, and the yield ratio are shown in Table 1.

**Comparative Example 3 (CE.3)**

The test was carried out according to the method of Example 1, except that the solid acid catalyst was changed to USY-3 molecular sieve, and the amount of n-butanol was 19 g, and the amount of tetrahydrofuran was 1 g. The reaction product liquid from the first step was analyzed to obtain the fructose conversion and the yields of the target products 5-hydroxymethylfurfural (HMF) and 5-(C2-C5 alkoxymethyl)furfural (RMF). The fructose conversion, the sum of HMF and RMF yields, and the yield ratio are shown in Table 1.

**Comparative Example 4 (CE.4)**

The test was carried out according to the method of Example 1, except that solvent I and solvent II were replaced with 20 g of n-butanol. The reaction product liquid from the first step was analyzed to obtain the fructose conversion and the yields of 5-hydroxymethylfurfural (HMF) and 5-(C2-C5 alkoxymethyl)furfural (RMF). The fructose conversion, the sum of HMF and RMF yields, and the yield ratio are shown in Table 1.

Table 1. Reaction Conditions and Results of Examples 1-16 and Comparative Examples 1-4

| Ex. | Bioma ss feedsto ck | Solid Acid Catalys t | Mass Ratio of Feedstoc k/ Catalyst | Organic Solvent | Mass Ratio Solvent I/Solvent II | Reactio n Temper ature (°C) | Reaction Time (min) | Feedsto ck Convers ion (%) | HMF+R MF yield (%) | HMF/R MF (mol%) |
|---|---|---|---|---|---|---|---|---|---|---|
| 1 | Fructo se | Prep. Ex. 1 | 8 | n-Butanol + THF | 1 | 165 | 30 | >99 | 71.5 | 0.85 |
| 2 | Fructo se | USY-3 | 8 | n-Butanol + THF | 1 | 165 | 30 | >99 | 62.7 | 0.80 |
| 3 | Fructo se | A-15 Resin | 8 | n-Butanol + THF | 1 | 165 | 30 | >99 | 60.3 | 1.25 |
| 4 | Fructo se | Prep. Ex. 1 | 12 | n-Butanol + THF | 1 | 165 | 30 | >99 | 70.1 | 0.84 |
| 5 | Fructo se | Prep. Ex. 1 | 8 | n-Butanol + THF | 1.5 | 165 | 30 | >99 | 69.5 | 0.79 |
| 6 | Fructo se | Prep. Ex. 1 | 8 | n-Butanol + THF | 2 | 165 | 30 | >99 | 68.3 | 0.68 |
| 7 | Fructo se | Prep. Ex. 1 | 8 | n-Butanol + THF | 0.75 | 165 | 30 | >99 | 67.2 | 1.05 |
| 8 | Fructo se | Prep. Ex. 1 | 8 | n-Butanol + THF | 0.5 | 165 | 30 | >99 | 61.9 | 1.35 |
| 9 | Fructo se | Prep. Ex. 1 | 8 | n-Butanol + THF | 1 | 150 | 30 | >99 | 60.7 | 0.81 |
| 10 | Fructo se | Prep. Ex. 1 | 8 | n-Butanol + THF | 1 | 180 | 30 | >99 | 62.2 | 0.72 |
| 11 | Fructo se | Prep. Ex. 1 | 8 | n-Butanol + THF | 1 | 165 | 20 | >99 | 67.5 | 0.79 |
| 12 | Fructo se | Prep. Ex. 1 | 8 | n-Butanol + THF | 1 | 165 | 50 | >99 | 59.8 | 0.65 |
| 13 | Glucos e | Prep. Ex. 1 | 8 | n-Butanol + THF | 1 | 165 | 30 | >99 | 50.7 | 0.75 |
| 14 | Fructo se | Prep. Ex. 1 | 8 | Ethanol + THF | 1 | 165 | 30 | >99 | 66.9 | 0.71 |
| 15 | Fructose | Prep. Ex. 1 | 8 | n-Butanol + MIBK | 1 | 165 | 30 | >99 | 65.6 | 0.95 |
| 16 | Fructo se | Prep. Ex. 1 | 8 | n-Butanol + 1,4-Di- oxane | 1 | 165 | 30 | 98.5 | 63.9 | 1.15 |
| CE.1 | Fructo se | Prep. Ex. 1 | 8 | Water | / | 165 | 30 | >99 | 25 | / |
| CE.2 | Fructo se | Prep. Ex. 1 | 8 | n-Butanol + Water | 1 | 165 | 30 | >99 | 48.1 | 0.19 |
| CE.3 | Fructo se | USY-3 | 8 | n-Butanol + THF | 19 | 165 | 30 | >99 | 43.2 | 0.27 |
| CE.4 | Fructo se | Prep. Ex. 1 | 8 | n-Butanol | / | 165 | 30 | >99 | 41.3 | 0.24 |

**Example 17**

0.2 g of the Ni-SCM-14 catalyst from Preparation Example 2 above and 15 g of the reaction product liquid obtained in Example 1, after filtration to remove the solid acid catalyst, were directly added as feedstock to an autoclave with stirrer, and 1.5 MPa of hydrogen was charged. The temperature was raised to the preset temperature using a programmed heating mantle, and stirring was performed with a magnetic stirrer. The reaction was carried out at a temperature of 190°C for a time of 3 h. The reaction product liquid from the second step was analyzed by gas chromatography to calculate the HMF+RMF conversion and the selectivity for the product DMF. The results are shown in Table 2.

**Example 18**

The test was carried out according to the method of Example 17, except that the hydrogenolysis catalyst was replaced with the Ni-SCM-15 catalyst from Preparation Example 3. The reaction product liquid from the second step was analyzed by gas chromatography to calculate the HMF+RMF conversion and the selectivity for the product DMF. The results are shown in Table 2.

**Example 19**

The test was carried out according to the method of Example 17, except that the hydrogenolysis catalyst was replaced with the Ni-$C_3N_4$/HC catalyst from Preparation Example 4. The reaction product liquid from the second step was analyzed by gas chromatography to calculate the HMF+RMF conversion and the selectivity for the product DMF. The results are shown in Table 2.

**Example 20**

The test was carried out according to the method of Example 17, except that the hydrogenolysis catalyst was replaced with a Ni-Fe/C catalyst (synthesized according to Przydacz M. Energies 2020, 13, 4660, with the support $TiO_2$ replaced with activated carbon, Ni content: 5 wt%, Fe content: 5 wt%). The reaction product liquid from the second step was analyzed by gas chromatography to calculate the HMF+RMF conversion and the selectivity for the product DMF. The results are shown in Table 2.

**Example 21**

The test was carried out according to the method of Example 17, except that the hydrogenolysis catalyst was replaced with a Pd/C (Acros Organics 195020100, 5% Pd) catalyst. The reaction product liquid from the second step was analyzed by gas chromatography to calculate the HMF+RMF conversion and the selectivity for the product DMF. The results are shown in Table 2.

**Example 22**

The test was carried out according to the method of Example 17, except that the hydrogenolysis catalyst was replaced with a Ru/C (Alfa Aesar, 044338, 5% Ru) catalyst. The reaction product liquid from the second step was analyzed by gas chromatography to calculate the HMF+RMF conversion and the selectivity for the product DMF. The results are shown in Table 2.

**Example 23**

The test was carried out according to the method of Example 17, except that the mass ratio of the reaction product liquid (15 g) obtained in Example 1 to the hydrogenolysis catalyst was 100. The reaction product liquid from the second step was analyzed by gas chromatography to calculate the HMF+RMF conversion and the selectivity for the product DMF. The results are shown in Table 2.

**Example 24**

The test was carried out according to the method of Example 17, except that the mass ratio of the reaction product liquid (15 g) obtained in Example 1 to the hydrogenolysis catalyst was 50. The reaction product liquid from the second step was analyzed by gas chromatography to calculate the HMF+RMF conversion and the selectivity for the product DMF. The

results are shown in Table 2.

**Example 25**

The test was carried out according to the method of Example 17, except that the reaction temperature was 180°C. The reaction product liquid from the second step was analyzed by gas chromatography to calculate the HMF+RMF conversion and the selectivity for the product DMF. The results are shown in Table 2.

**Example 26**

The test was carried out according to the method of Example 17, except that the reaction temperature was 200°C. The reaction product liquid from the second step was analyzed by gas chromatography to calculate the HMF+RMF conversion and the selectivity for the product DMF. The results are shown in Table 2.

**Example 27**

The test was carried out according to the method of Example 17, except that the reaction time was 2.8 h. The reaction product liquid from the second step was analyzed by gas chromatography to calculate the HMF+RMF conversion and the selectivity for the product DMF. The results are shown in Table 2.

**Example 28**

The test was carried out according to the method of Example 17, except that the reaction time was 3.2 h. The reaction product liquid from the second step was analyzed by gas chromatography to calculate the HMF+RMF conversion and the selectivity for the product DMF. The results are shown in Table 2.

**Example 29**

The test was carried out according to the method of Example 17, except that the reaction product liquid obtained in Example 1 was replaced with the reaction product liquid obtained in Example 2. The reaction product liquid from the second step was analyzed by gas chromatography to calculate the HMF+RMF conversion and the selectivity for the product DMF. The results are shown in Table 2.

**Example 30**

The test was carried out according to the method of Example 17, except that the reaction product liquid obtained in Example 1 was replaced with the reaction product liquid obtained in Example 8. The reaction product liquid from the second step was analyzed by gas chromatography to calculate the HMF+RMF conversion and the selectivity for the product DMF. The results are shown in Table 2.

**Example 31**

The test was carried out according to the method of Example 17, except that the reaction product liquid obtained in Example 1 was replaced with the reaction product liquid obtained in Example 14. The reaction product liquid from the second step was analyzed by gas chromatography to calculate the HMF+RMF conversion and the selectivity for the product DMF. The results are shown in Table 2.

**Example 32**

The test was carried out according to the method of Example 17, except that the reaction product liquid obtained in Example 1 was replaced with the reaction product liquid obtained in Example 16. The reaction product liquid from the second step was analyzed by gas chromatography to calculate the HMF+RMF conversion and the selectivity for the product DMF. The results are shown in Table 2.

**Comparative Example 5 (CE.5)**

The test was carried out according to the method of Example 17, except that the reaction product liquid obtained in Example 1 was replaced with the reaction product liquid obtained in Comparative Example 1. The reaction product liquid

from the second step was analyzed by gas chromatography to calculate the HMF+RMF conversion and the selectivity for the product DMF. The results are shown in Table 2.

**Comparative Example 6 (CE.6)**

The test was carried out according to the method of Example 17, except that the reaction product liquid obtained in Example 1 was replaced with the reaction product liquid obtained in Comparative Example 2. The reaction product liquid from the second step was analyzed by gas chromatography to calculate the HMF+RMF conversion and the selectivity for the product DMF. The results are shown in Table 2.

**Comparative Example 7 (CE.7)**

The test was carried out according to the method of Example 17, except that the reaction product liquid obtained in Example 1 was replaced with the reaction product liquid obtained in Comparative Example 3. The reaction product liquid from the second step was analyzed by gas chromatography to calculate the HMF+RMF conversion and the selectivity for the product DMF. The results are shown in Table 2.

**Comparative Example 8 (CE.8)**

The test was carried out according to the method of Example 17, except that the reaction product liquid obtained in Example 1 was replaced with the reaction product liquid obtained in Comparative Example 4. The reaction product liquid from the second step was analyzed by gas chromatography to calculate the HMF+RMF conversion and the selectivity for the product DMF. The results are shown in Table 2.

Table 2. Reaction Conditions and Results of Examples 17-32 and Comparative Examples 5-8

| Ex. | Hydrogenolysis Catalyst | Source of Reaction Product liquid (Feedstock) | HMF/RMF (Feedstock) | Mass Ratio of Feedstock to Catalyst | Hydrogen Pressure (MPa) | Reaction Temperature (°C) | Reaction Time (min) | HMF+RMF Conversion (%) | DMF Selectivity (%) |
|---|---|---|---|---|---|---|---|---|---|
| 17 | Prep. Ex. 2 | EX.1 | 0.85 | 75 | 1.5 | 190 | 3 | >99 | 80.5 |
| 18 | Prep. Ex. 3 | EX.1 | 0.85 | 75 | 1.5 | 190 | 3 | >99 | 75.2 |
| 19 | Prep. Ex. 4 | EX.1 | 0.85 | 75 | 1.5 | 190 | 3 | >99 | 81.5 |
| 20 | Ni-Fe/C | EX.1 | 0.85 | 75 | 1.5 | 190 | 3 | 95.0 | 71.2 |
| 21 | Pd/C | EX.1 | 0.85 | 75 | 1.5 | 190 | 3 | >99 | 70.1 |
| 22 | Ru/C | EX.1 | 0.85 | 75 | 1.5 | 190 | 3 | >99 | 71.2 |
| 23 | Prep. Ex. 2 | EX.1 | 0.85 | 100 | 1.5 | 190 | 3 | 98.1 | 77.2 |
| 24 | Prep. Ex. 2 | EX.1 | 0.85 | 50 | 1.5 | 190 | 3 | >99 | 73.7 |
| 25 | Prep. Ex. 2 | EX.1 | 0.85 | 75 | 1.5 | 180 | 3 | 98.6 | 70.9 |
| 26 | Prep. Ex. 2 | EX.1 | 0.85 | 75 | 1.5 | 200 | 3 | >99 | 71.8 |
| 27 | Prep. Ex. 2 | EX.1 | 0.85 | 75 | 1.5 | 190 | 2.8 | 97.3 | 68.6 |
| 28 | Prep. Ex. 2 | EX.1 | 0.85 | 75 | 1.5 | 190 | 3.2 | >99 | 69.8 |
| 29 | Prep. Ex. 2 | EX.2 | 0.80 | 75 | 1.5 | 190 | 3 | >99 | 69.1 |
| 30 | Prep. Ex. 2 | EX.8 | 1.35 | 75 | 1.5 | 190 | 3 | >99 | 68.3 |
| 31 | Prep. Ex. 2 | EX.14 | 0.71 | 75 | 1.5 | 190 | 3 | >99 | 75.1 |
| 32 | Prep. Ex. 2 | EX.16 | 1.15 | 75 | 1.5 | 190 | 3 | 98.3 | 71.8 |
| CE. 5 | Prep. Ex. 2 | CE. 1 | / | 75 | 1.5 | 190 | 3 | 67.2 | 33.0 |
| CE. 6 | Prep. Ex. 2 | CE. 2 | 0.19 | 75 | 1.5 | 190 | 3 | 80.3 | 56.1 |
| CE. 7 | Prep. Ex. 2 | CE. 3 | 0.27 | 75 | 1.5 | 190 | 3 | 99 | 40.0 |

(continued)

| Ex. | Hydrogenolysis Catalyst | Source of Reaction Product liquid (Feedstock) | HMF/RMF (Feedstock) | Mass Ratio of Feedstock to Catalyst | Hydrogen Pressure (MPa) | Reaction Temperature (°C) | Reaction Time (min) | HMF+ RMF Conversion (%) | DMF Selectivity (%) |
|---|---|---|---|---|---|---|---|---|---|
| CE. 8 | Prep. Ex. 2 | CE. 4 | 0.24 | 75 | 1.5 | 190 | 3 | 80.6 | 52.3 |

The specific embodiments of the present disclosure have been described in detail above, however, the present disclosure is not limited thereto. Within the technical concept of the present disclosure, various simple modifications can be made to the technical solution of the present disclosure, including combining various technical features in any other suitable manner. These simple modifications and combinations should also be regarded as the content disclosed in the present disclosure and fall within the protection scope of the present disclosure.

## Claims

**1.** A method for preparing 2,5-dimethylfuran by biomass conversion, comprising:

(1) in an organic solvent, subjecting a biomass feedstock to a reaction in the presence of a solid acid catalyst to obtain a reaction product liquid containing 5-hydroxymethylfurfural and 5-(C2-C5 alkoxymethyl)furfural, wherein the molar ratio of 5-hydroxymethylfurfural to 5-(C2-C5 alkoxymethyl)furfural is in a range of 0.5-1.5;
(2) subjecting the reaction product liquid obtained in step (1) and hydrogen to a reaction in the presence of a hydrogenolysis catalyst to prepare 2,5-dimethylfuran.

**2.** The method according to claim 1, **characterized in that**, in step (1), the organic solvent comprises solvent I and solvent II; the solvent I is selected from at least one of C2-C5 alcohols, preferably selected from at least one of ethanol, n-propanol, n-butanol, isobutanol, isopropanol, isoamyl alcohol, ethylene glycol, propylene glycol and glycerol, more preferably selected from one or two of isopropanol, isoamyl alcohol and n-butanol; the solvent II is selected from at least one of cyclic ether compounds, methyl isobutyl ketone and $\gamma$-valerolactone, the cyclic ether compound is preferably selected from at least one of pyran, 2-methyltetrahydrofuran, 2,5-dimethyltetrahydrofuran, tetrahydrofuran and 1,4-dioxane, and the solvent II is preferably selected from one or two of 2,5-dimethyltetrahydrofuran, 1,4-dioxane and tetrahydrofuran;

preferably, the mass ratio of the solvent I to the solvent II is in a range of 0.1-15:1, preferably 0.5-10:1; and/or, preferably, the mass ratio of the total amount of the organic solvent to the biomass feedstock is in a range of 1-100:1, preferably 2-50:1.

**3.** The method according to any one of claims 1-2, **characterized in that**, in step (1), the biomass feedstock is a biomass feedstock containing hexose and/or a biomass feedstock that produces hexose upon treatment, the hexose being selected from one or more of galactose, mannose, glucose and fructose, preferably fructose and/or glucose; and/or, in step (1), the mass ratio of the biomass feedstock to the solid acid catalyst is in a range of 0.2-20: 1, preferably 0.4-20:1.

**4.** The method according to any one of claims 1-3, **characterized in that**, in step (1), the reaction temperature is in a range of 90-220°C, preferably 120-200°C; the reaction time is in a range of 5-300 min, preferably 10-240 minutes; and/or,
the reaction in step (1) is carried out under an inert gas, and the inert gas pressure is in a range of 0.1-3.0 MPa, preferably 0.5-2.0 MPa.

**5.** The method according to any one of claims 1-4, **characterized in that**, in step (1), the solid acid catalyst is selected from one or more of SCM-36-$SO_3H$ molecular sieve, SCM-1-$SO_3H$ molecular sieve, H-beta molecular sieve, USY molecular sieve, SBA-15-$SO_3H$ molecular sieve, sulfonated carbon, A-15 resin or Aquivion@$SiO_2$ resin; preferably SCM-36-$SO_3H$ molecular sieve;

preferably, the Bronsted acid/Lewis acid ratio of the SCM-36-$SO_3H$ molecular sieve is in a range of 2.0-4.5,

preferably 2.2-4.0; and/or,
preferably, in the SCM-36-$SO_3H$ molecular sieve, the content of -$SO_3H$ is in a range of 1.5 wt%-20.0 wt%, preferably 1.9 wt%-18.0 wt%; and/or,
preferably, the external specific surface area/total specific surface area ratio of the SCM-36-$SO_3H$ molecular sieve is in a range of 0.25-0.9, preferably 0.25-0.7.

**6.** The method according to any one of claims 1-5, **characterized in that**, in step (2), the hydrogenolysis catalyst is a supported noble metal and/or nonnoble metal catalyst, preferably selected from one or more of Pd/C, Pt/C, Ru/C, Pd/$Al_2O_3$, Pt/$Al_2O_3$, Ru/$Al_2O_3$, Ru/$Co_3O_4$, Ni-$Co_3O_4$/C, Ni/$Al_2O_3$, Ni-Fe/C, Ni-$C_3N_4$/HC catalyst and Ni-SCM-X molecular sieve catalyst; more preferably selected from Ni-SCM-X molecular sieve catalyst and Ni-$C_3N_4$/HC catalyst.

**7.** The method according to claim 6, **characterized in that**, in the Ni-SCM-X molecular sieve catalyst, X is 14 or 15; and/or,

in the Ni-SCM-X molecular sieve catalyst, Ni comprises metallic Ni and $Ni^{2+}$, wherein the molar ratio of Ni/$Ni^{2+}$ is in a range of 0.02-0.15, preferably 0.05-0.12; and/or,
in the Ni-SCM-X molecular sieve catalyst, Ni is present as nickel particles, wherein the mean particle size of the nickel particles is in a range of 2.0-8.0 nm, preferably 3.0-7.0 nm, more preferably 3.0-6.0 nm; and/or,
in the Ni-SCM-X molecular sieve catalyst, based on the mass of the SCM-X molecular sieve, the content of Ni, calculated as elemental Ni, is in a range of 0.2 wt%-5.0 wt%, preferably 0.5 wt%-3.0 wt%; and/or,
in the Ni-SCM-X molecular sieve catalyst, the Si/Ge molar ratio of the SCM-X molecular sieve is in a range of 1.5-8, preferably 2-7.

**8.** The method according to claim 6 or 7, **characterized in that**, the total acid amount of the Ni-SCM-X molecular sieve catalyst is in a range of 130-220 $\mu mol \cdot g^{-1}$, preferably 150-200 $\mu mol \cdot g^{-1}$; and/or,

the Bronsted acid/Lewis acid ratio of the Ni-SCM-X molecular sieve catalyst is in a range of 0.3-0.8, preferably 0.4-0.6; and/or,
the total specific surface area of the Ni-SCM-X molecular sieve catalyst is in a range of 90-370 $m^2 \cdot g^{-1}$, preferably 100-350 $m^2 \cdot g^{-1}$.

**9.** The method according to claim 6, **characterized in that**, the Ni-$C_3N_4$/HC catalyst comprises nickel, $C_3N_4$, and a support, wherein the support is activated carbon activated with hydrogen (HC).

**10.** The method according to claim 9, **characterized in that**, in the Ni-$C_3N_4$/HC catalyst, based on the mass of the support, the content of nickel, calculated as elemental Ni, is in a range of 0.5 wt%-10 wt%, and the content of $C_3N_4$ is in a range of 2 wt%-30 wt%; preferably, the content of nickel, calculated as elemental Ni, is in a range of 1 wt%-6 wt%, and the content of $C_3N_4$ is in a range of 4 wt%-20 wt%; and/or

the oxygen content of the HC is in a range of 4 wt%-18 wt%, preferably 6 wt%-15 wt%; and/or
the total specific surface area of the Ni-$C_3N_4$/HC catalyst is in a range of 700-1800 $m^2 \cdot g^{-1}$, preferably 800-1300 $m^2 \cdot g^{-1}$; and/or
the Ni-$C_3N_4$/HC catalyst comprises Ni particles encapsulated by graphitic $C_3N_4$ shell; and/or
the molar ratio of $Ni_3N$ species to the oxidized NiO in the Ni-$C_3N_4$/HC catalyst is in a range of 0.25-0.60:1, preferably 0.30-0.50:1.

**11.** The method according to any one of claims 1-10, **characterized in that**, in step (2), the mass ratio of the reaction product liquid containing 5-hydroxymethylfurfural and 5-(C2-C5 alkoxymethyl)furfural obtained in step (1) to the hydrogenolysis catalyst is in a range of 30-200:1, preferably 50-150:1.

**12.** The method according to any one of claims 1-11, **characterized in that** the reaction in step (2) is carried out under the following conditions:

the reaction in step (2) is carried out under a hydrogen atmosphere, and the hydrogen pressure is in a range of 0.5-4.0 MPa, preferably 1-3.0 MPa; and/or,
the reaction temperature is in a range of 130-220°C, preferably 150-200°C; the reaction time is in a range of 1-12 h, preferably 2-10 h.

13. The method according to any one of claims 1-12, wherein the step (1) further comprises filtering a mixture produced by the reaction to remove the solid acid catalyst to obtain the reaction product liquid, preferably the reaction product liquid obtained in step (1) is directly used in step (2).

Biomass Feedstock
Solvent I
Solvent II
Acid Catalyst
Solvent Recycling
Catalyst Regeneration and Recycling
Hydrogenolysis
Catalyst Loading
Bio-Based
2,5-Dimethylfuran
(DMF)
1
2
3
4
1、Tank Reactor 2、Solid-Liquid Separator 3、Fixed-Bed Reactor 4、Distillation Unit

Fig.1

3739 cm-1
Hydrogen-Form C-FER
Hydrogen-Form SCM-36
Absorbance (a.u.)
Wavenumber(cm-1)
0.70
0.65
0.60
0.55
0.50
0.45
0.40
0.35
0.30
0.25
3900
3800
3700
3600
3500
3400
3300

Fig.2

SCM-36-SO3H
Intensity (a.u.)
2θ (°)
10
20
30
40
50
60
70

Fig.3

3.0kV x50.0k SE(U)
1.00um

Fig.4

SCM-36-SO3H
Absorbance (a.u.)
1400
1450
1500
1550
1600
Wavenumber (cm-1)

Fig.5

Ni-SCM-14
Intensity (a.u.)
10
20
30
40
50
60
70
2θ(°)

Fig.6

Ni-SCM-14
Intensity (a.u.)
100
200
300
400
500
Bed Temperature (°C)

Fig.7

Ni 2p3/2
Ni2+ 93.0%
855.6
Ni0 7.0%
852.0
Intensity (a.u.)
868
866
864
862
860
858
856
854
852
850
Binding Energy (eV)

Fig.8

50 nm

Mean Particle Size
3.48±0.55 nm
2
2.5
3
3.5
4
4.5
5
5.5
Particle Size (nm)

Fig.9

Ni-SCM-15
Intensity (a.u.)
0
10
20
30
40
50
2θ(°)

Fig.10

Intensity (a.u.)
0
10
20
30
40
50
60
70
2θ(°)

Fig.11

NiO(1 0 1)
0.242 nm
Ni3N(1 1 0)
20 nm


Fig.12

(a)
Ni 2p3/2
Ni2+
75.7%
Sat.
Ni3N
24.3%
Intensity (a.u.)
868
864
860
856
852
Binding Energy (eV)


Fig.13

## INTERNATIONAL SEARCH REPORT

International application No.
**PCT/CN2024/122732**

**A. CLASSIFICATION OF SUBJECT MATTER**

B01J29/072(2006.01)i

According to International Patent Classification (IPC) or to both national classification and IPC

**B. FIELDS SEARCHED**

Minimum documentation searched (classification system followed by classification symbols)

B01J

Documentation searched other than minimum documentation to the extent that such documents are included in the fields searched

Electronic data base consulted during the international search (name of data base and, where practicable, search terms used)

CNTXT, VEN, CJFD: 生物质, 二甲基呋喃, 羟甲基糠醛, 加氢, 催化剂, 固体酸, biomass, DMF, HMF, hydrogenation, catalyst, solid acid

**C. DOCUMENTS CONSIDERED TO BE RELEVANT**

| Category* | Citation of document, with indication, where appropriate, of the relevant passages | Relevant to claim No. |
|---|---|---|
| X | US 2016304480 A1 (KOREA RESEARCH INSTITUTE OF CHEMICAL TECHNOLOGY) 20 October 2016 (2016-10-20) description, paragraphs 14-47 | 1-13 |
| A | CN 111875566 A (HUNAN NORMAL UNIVERSITY) 03 November 2020 (2020-11-03) entire document | 1-13 |
| A | JP 2018090511 A (NATIONAL INSTITUTE OF ADVANCED INDUSTRIAL SCIENCE AND TECHNOLOGY et al.) 14 June 2018 (2018-06-14) entire document | 1-13 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2024** | **03 January 2025** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/122732**

| Patent document cited in search report | Publication date (day/month/year) | Patent family member(s) | Publication date (day/month/year) |
|---|---|---|---|
| US 2016304480 A1 | 20 October 2016 | US 9988362 B2 | 05 June 2018 |
| | | KR 20160123072 A | 25 October 2016 |
| | | KR 101767182 B1 | 11 August 2017 |
| CN 111875566 A | 03 November 2020 | None | |
| JP 2018090511 A | 14 June 2018 | JP 2022000446 A | 04 January 2022 |
| | | JP 7264961 B2 | 25 April 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103554066 A
- CN 105175366 A
- CN 115959681 A
- CN 109081360 A
- WO 2018227849 A1
- CN 109081359 A
- WO 2018227850 A1


### Non-patent literature cited in the description


- *Appl. Catal. B: Environ. [J].*, 2014, vol. 146, 244-248
- *Chinese Journal of Catalysis [J].*, 2024, vol. 60, 253-261